# EUROPEAN PATENT SPECIFICATION

(11) **EP 0 539 420 B1**
(45) Date of publication and mention of the grant of the patent: **05.01.2000**
(21) Application number: 91912667.2
(22) Date of filing: 09.07.1991
(51) Int. Cl.: C07D 513/04, C07D 401/14, C07D 401/04, C07D 519/00, C07D 473/40, A61K 31/505, A61K 31/425, C07D 417/14, C07D 471/04, C07D 473/00, C07D 473/30

(54) **SUBSTITUTED THIAZOLYL AND SUBSTITUTED PYRIDINYL DERIVATIVES**
SUBSTITUIERTE THIAZOL- UND PYRIDINDERIVATE
DERIVES DE THIAZOLYLE SUBSTITUE ET DE PYRIDINYLE SUBSTITUE

(30) Priority: 19.07.1990 US 554325
(43) Date of publication of application: 05.05.1993
(73) Proprietor: JANSSEN PHARMACEUTICA N.V., 2340 Beerse (BE)
(72) Inventor: JANSSENS, Frans, Eduard, B-2820 Bonheiden (BE); SOMMEN, François, Maria, B-2323 Wortel (BE); DIELS, Gaston, Stanislas, Marcella, B-2380 Ravels (BE)
(74) Representative: Wante, Dirk
(86) International application number: EP9101292
(87) International publication number: WO9201697

(56) References cited:
- EP-A- 0 206 415
- EP-A- 0 297 661
- US-A- 4 556 660
- US-A- 4 588 722
- US-A- 4 634 704
- US-A- 4 695 569
- US-A- 4 695 575
- US-A- 4 835 161
- US-A- 4 897 401

## Description

### Background of the invention

In US-A-4,556,660; US-A-4,588,722 and US-A-4,897,401 there are disclosed N-(benzimidazole and imidazopyridine)-4-piperidinamines.

In US-A-4,695,569 there are disclosed bicyclic heterocyclyl containing N-(benzimidazole and imidazopyridine)-4-piperidinamines.

In US-A-4,695,575 and US-A-4,634,704 there are disclosed benzimidazole and imidazopyridine substituted piperidine derivatives.

In US-A-4,835,161 there are disclosed N-(benzimidazole and imidazopyridine)-4-piperidinamines wherein the benzimidazole or imidazopyridine moiety is substituted with a alkyl substituted furanyl moiety.

In EP-A-0,206,415 there are disclosed purine substituted piperidine derivatives.

In EP-A-0,297,661 there are disclosed benzimidazole and imidazopyridine substituted pyrrolidine- and homopiperidine derivatives.

The above art-known compounds are all described as antihistaminics and serotonin antagonists.

### Description of the invention:

The present invention is concerned with substituted thiazolyl and substituted pyridinyl derivatives having the formula : 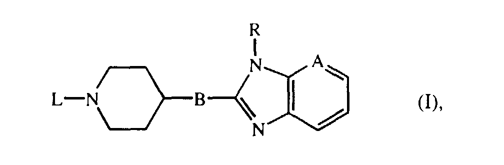 the pharmaceutically acceptable acid addition salts and the stereochemically isomeric forms thereof, wherein
- -A=: is -N= or -CH=;
- B: represents NH or CH₂;
- R: is a radical of formula; 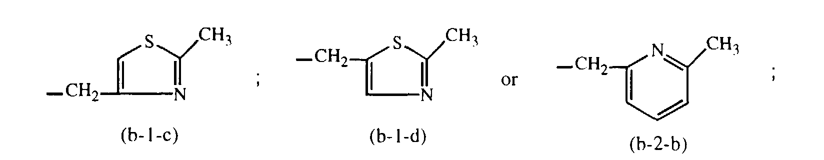
- L: is hydrogen; C₁₋₁₂alkyl; C₁₋₆alkyloxycarbonyl; or a radical of formula

-Alk-R³ (c-1);

-Alk-Y-R⁴ (c-2);

or

-Alk-Z¹-C(=O)-Z²-R⁵ (c-3);

wherein
- R³: is Het or phenyl optionally substituted with halo, C₁₋₆alkyl, hydroxy or C₁₋₆alkyloxy;
- R⁴: is hydrogen, Het; C₁₋₆alkyl or phenyl optionally substituted with halo, C₁₋₆alkyl, hydroxy or C₁₋₆alkyloxy;
- R⁵: is hydrogen, Het, C₁₋₆alkyl or phenyl optionally substituted with halo, C₁₋₆alkyl, hydroxy or C₁₋₆alkyloxy;
- Y: is O or NH;
Z¹ and Z² each independently are O or NH;
each Alk independently is C₁₋₆alkanediyl;

Het is pyridinyl, optionally substituted with one or two substituents each independently selected from halo, amino, mono- and di(C₁₋₆alkyl)amino, aryl-C₁₋₆alkylamino, nitro, cyano, aminocarbonyl, C₁₋₆alkyl, C₁₋₆alkyloxy, C₁₋₆alkylthio, C₁₋₆alkyloxycarbonyl, hydroxy, C₁₋₆alkylcarbonyloxy, arylC₁₋₆alkyl and carboxyl; pyridinyloxide, optionally substi-tuted with nitro; pyrimidinyl, optionally substituted with one or two substituents each independently selected from halo, amino, C₁₋₆alkylamino, arylC₁₋₆alkylamino, hydroxy, C₁₋₆alkyl, C₁₋₆alkyloxy, C₁₋₆alkylthio and arylC₁₋₆alkyl; pyridazinyl, optionally substituted with C₁₋₆alkyl or halo; pyrazinyl, optionally substituted with halo, amino or C₁₋₆alkyl; thienyl, optionally substituted with halo or C₁₋₆alkyl; furanyl, optionally substituted with halo or C₁₋₆alkyl; pyrrolyl, optionally substituted with C₁₋₆alkyl; thiazolyl, optionally substituted with C₁₋₆alkyl, C₁₋₆alkyloxycarbonyl, aryl or arylC₁₋₆alkyl; imidazolyl, optionally substituted with one or two substituents each independently selected from C₁₋₆alkyl, arylC₁₋₆alkyl and nitro; tetrazolyl, optionally substituted with C₁₋₆alkyl; 1,3,4-thiadiazolyl, optionally substituted with C₁₋₆alkyl or amino; 5,6-dihydro-4H-1,3-thiazin-2-yl, optionally substituted with C₁₋₆alkyl; 4,5-di-hydrothiazolyl, optionally substituted with C₁₋₆alkyl; oxazolyl, optionally substituted with C₁₋₆alkyl; 4,5-dihydro-5-oxo-1H-tetrazolyl, optionally substituted with C₁₋₆alkyl; 1,4-dihydro-2,4-dioxo-3(2H)-pyrimidinyl, optionally substituted with C₁₋₆alkyl; 3,4-dihydro-4-oxopyrimidinyl or 4,5-dihydro-4-oxopyrimidinyl, both radicals optionally substituted with up to 3 substituents selected from C₁₋₆alkyl, amino, C₁₋₆alkylaminocarbonylamino, arylaminocarbonylamino, arylC₁₋₆alkylamino and C₁₋₆alkylamino; 2,3-dihydro-3-oxopyridazinyl; 2-oxo-3-oxazolidinyl; pyrrolidinyl; piperidinyl; morpholinyl; thiomorpholinyl; dioxanyl, optionally substituted with C₁₋₆alkyl; indolyl, optionally substituted with hydroxy or C₁₋₆alkyl; quinolinyl, optionally substituted with hydroxy or C₁₋₆alkyl; quinazolinyl, optionally substituted with hydroxy or C₁₋₆alkyl; quinoxalinyl, optionally substituted with C₁₋₆alkyl; phthala-zinyl, optionally substituted with halo; 1,3-dioxo-1H-isoindol-2(3H)-yl; 2,3-dihydro-3-oxo-4H-benzoxazinyl and 2,3-dihydro-1,4-benzodioxinyl, both being optionally substituted with C₁₋₆alkyl or halo; 2-oxo-2H-1-benzopyranyl and 4-oxo-4H-1-benzo-pyranyl. both being optionally substituted with C₁₋₆alkyl; 3,7-dihydro-1,3-dimethyl-2,6-dioxo-1H-purin-7-yl, optionally substituted with C₁₋₆alkyl; 6-purinyl, and
a bicyclic heterocyclic radical of formula 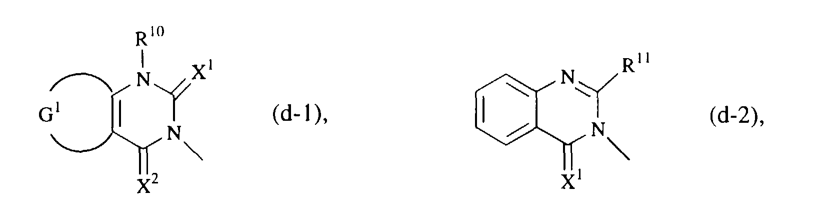 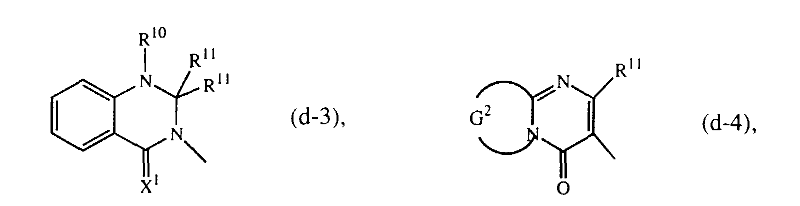 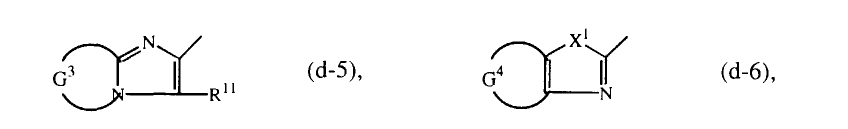 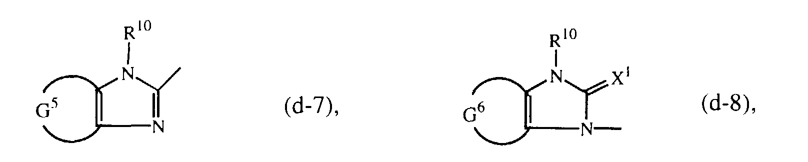 wherein
X¹ and X² each independently are O or S ;
each R¹⁰ and R¹¹ independently are hydrogen or C₁₋₆alkyl;

- G¹: is -CH=CH-CH=CH-; -S-CH=CH- or -N=CH-NH- ;
- G²: is -CH=CH-CH=CH-, -(CH₂)₄-, -S-(CH₂)₂-, -S-(CH₂)₃-, -S-CH=CH-, -CH=CH-O-, -NH-(CH₂)₂-, -NH-(CH₂)₃-, -NH-CH=CH-, -NH-N=CH-CH₂-, -NH-CH=N- or -NH-N=CH- ;
- G³: is -CH=CH-CH=CH-, -CH₂-NH-(CH₂)₂-, -S-CH=CH-, -S-(CH₂)₃-, -N=CH-CH=CH-, -CH=N-CH=CH-, -CH=CH-N=CH-, -CH=CH-CH=N-, -N=CH-N=CH- or -CH=N-CH=N- ;
- G⁴: is -CH=CH-CH=CH-, -CH₂-NH-(CH₂)₂-, -N=CH-CH=CH-, -CH=N-CH=CH-, -CH=CH-N=CH-, -CH=CH-CH=N-, -N=CH-N=CH- or -CH=N-CH=N-;
- G⁵: is -CH=CH-CH=CH-, -N=CH-CH=CH-, -CH=N-CH=CH-, -CH=CH-N=CH-, -CH=CH-CH=N-, -N=CH-N=CH- or -CH=N-CH=N- ;
- G⁶: is -CH=CH-CH=CH-, -N=CH-CH=CH-, -CH=N-CH=CH-, -CH=CH-N=CH-, -CH=CH-CH=N-, -N=CH-N=CH- or -CH=N-CH=N- .

As used in the foregoing definitions halo is generic to fluoro, chloro, bromo and iodo; C₁₋₄alkyl defines straight and branch chained saturated hydrocarbon radicals having from 1 to 4 carbon atoms such as, for example, methyl, ethyl, propyl, 1-methylethyl, butyl, 1,1-dimethylethyl, 1-methylpropyl, 2-methylpropyl; C₁₋₆alkyl defines C₁₋₄alkyl radicals as defined hereinabove and the higher homologs thereof having 5 or 6 carbon atoms; C₁₋₁₂ alkyl defines C₁₋₄ alkyl radicals as defined herein-above and the higher homologs thereof having from 5 to 12 carbon atoms; C₃₋₆cycloalkyl is generic to cyclopropyl, cyclobutyl, cyclopentyl and cyclohexyl; C₁₋₄alkanediyl defines bivalent straight and branch chained saturated hydrocarbon radicals having from 1 to 4 carbon atoms such as, for example, methylene, 1,2-ethanediyl, 1,3-propanediyl, 1,4-butanediyl and the branched isomers thereof; C₁₋₆alkanediyl defines C₁₋₄alkanediyl radicals as defined hereinabove and the higher homologs thereof having 5 or 6 carbon atoms such as, for example, 1,5-pentanediyl, 1,6-hexanediyl and the branched isomers thereof.

The pharmaceutically acceptable acid addition salts as mentioned hereinabove comprise the therapeutically active non-toxic acid addition salt forms which the compounds of formula (I) are able to form. Said salt forms can conveniently be obtained by treating the base form of the compounds of formula (I) with appropriate acids such as inorganic acids, for example, hydrohalic acid, e.g. hydrochloric and, hydrobromic acid, sulfuric acid, nitric acid, or phosphoric acid; or organic acids, such as, for example, acetic, propanoic, hydroxyacetic, 2-hydroxypropanoic, 2-oxopropanoic, ethanedioic, propanedioic, butanedioic, (Z)-2-butenedioic, (E)-2-butenedioic, 2-hydroxybutanedioic, 2,3-dihydroxybutanedioic, 2-hydroxy-1,2,3-propanetricarboxylic, methanesulfonic, ethanesulfonic, benzenesulfonic, 4-methylbenzenesulfonic, cyclohexanesulfamic, 2-hydroxybenzoic and, 4-amino-2-hydroxybenzoic acids. Conversely the salt form can be converted by treatment with alkali into the free base form.

The term acid addition salt also comprises the hydrates and solvent addition forms which the compounds of formula (I) are able to form. Examples of such forms are e.g. hydrates and, alcoholates.

The compounds of this invention may have several asymmetric carbon atoms in their structure. Each of these chiral centers may be indicated by the stereochemical descriptors R and S.

Pure stereochemically isomeric forms of the compounds of formula (I) may be obtained by the application of art-known procedures. Diastereoisomers may be separated by physical methods such as selective crystallization and chromatographic techniques, e.g. counter current distribution, liquid chromatography and the like; and enantiomers may be separated from each other following art-known resolution methods, for example, by the selective crystallization of their diastereomeric salts with chiral acids. Pure stereochemically isomeric forms may also be derived from the corresponding pure stereochemically isomeric forms of the appropriate starting materials, provided that the reactions occur stereospecifically. Preferably, if a specific stereoisomer is desired, said compound will be synthesized by stereoselective methods of preparation. These methods will advantageously employ enantiomerically pure starting materials. Stereochemically isomeric forms of the compounds of formula (I) are obviously intended to be included within the scope of the invention.

Preferred compounds are the compounds of formula (I) wherein L is hydrogen; C₁₋₆alkyl; a radical of formula (c-1) wherein R³ is phenyl optionally substituted with halo, C₁₋₆alkyl, hydroxy or C₁₋₆alkyloxy or Het; a radical of formula (c-2) wherein R⁴ is phenyl optionally substituted with halo. C₁₋₆alkyl, hydroxy or C₁₋₆alkyloxy or Het; or a radical of formula -Alk-NH-CO-Het (c-3-a); wherein each Het is pyridinyl, optionally substituted with amino or C₁₋₆alkyl; pyrimidinyl, optionally substituted with amino or C₁₋₆alkyl; pyrazinyl, optionally substituted with amino; thienyl; furanyl; thiazolyl, optionally substituted with C₁₋₆alkyl; imidazolyl, optionally substituted with C₁₋₆alkyl; tetrazolyl, optionally substituted with C₁₋₆alkyl; 1,3,4-thiadiazolyl, optionally substituted with C₁₋₆alkyl or amino; oxazolyl, optionally substituted with C₁₋₆alkyl; 4,5-dihydro-5-oxo-1H-tetrazolyl, optionally substituted with C₁₋₆alkyl; 1,4-dihydro-2,4-dioxo-3(2H)-pyrimidinyl; 3,4-dihydro-4-oxopyrimidinyl optionally substituted with up to 3 substituents selected from C₁₋₆alkyl, amino and C₁₋₆alkylamino; 2-oxo-3-oxazolidinyl; indolyl, optionally substituted with C₁₋₆alkyl; phthalazinyl; 2-oxo-2H-1-benzopyranyl; 3,7-dihydro-1,3-dimethyl-2,6-dioxo-1H-purin-7-yl, optionally substituted with C₁₋₆alkyl; 6-purinyl, or a bicyclic heterocyclic radical of formula (d-1) to (d-8) as defined hereinabove, wherein R¹⁰ and R¹¹ each independently are hydrogen or C₁₋₆alkyl and in the radicals (d-2) and (d-3), X¹ is O.

More preferred compounds are those preferred compounds wherein L is hydrogen or C₁₋₃alkyl.

Further more preferred compounds are those preferred compounds wherein L is a radical of formula -Alk-R³ (c-1) wherein R³ is 4-methoxyphenyl; 4-hydroxyphenyl; thienyl; thiazolyl optionally substituted with C₁₋₆alkyl; oxazolyl; 4,5-dihydro-1H-tetrazolyl optionally substituted with C₁₋₆alkyl; 2,3-dihydro-2-oxo-benzimidazol-1-yl; 1,4-dihydro-2,4-dioxo-3(2H)-pyrimidinyl; thienyl; 2-oxo-2H-1-benzopyranyl or R³ is a radical of formula 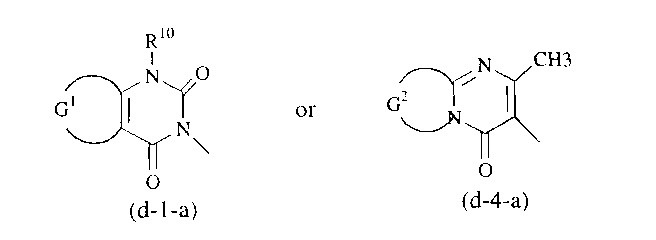 wherein G¹, G² and R¹⁰ arc as defined hereinabove.

Still other more preferred compounds are those preferred compounds wherein L is a radical of formula -Alk-Y-R⁴ (c-2) wherein R⁴ is thiazolyl, pyridinyl, 1,3,4-thiadiazolyl optionally substituted with C₁₋₆alkyl or amino, pyrimidinyl optionally substituted with amino, 6-purinyl, 3,4-dihydro-4-oxopyrimidinyl, phthalazinyl or 3H-imidazo[4,5-c]pyridin-2-yl.

Interesting compounds within the present invention are those compounds of formula (I) wherein L represents hydrogen, C₁₋₄alkyl, C₁₋₄alkyl-oxycarbonyl or a radical of formula -Alk-R³ (c-1), -Alk-Y-R⁴ (c-2) or -Alk-Z¹-C(=O)-Z²-R⁵ (c-3);
Alk represents C₁₋₄alkanediyl; R³ represents phenyl, C₁₋₄alkyloxyphenyl or a radical of formula 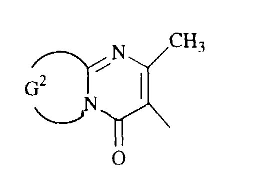 wherein G² represents -CH=CH-CH=CH-, -S-(CH₂)₃-, -S-(CH₂)₂- or -S-CH=CH-; Y represents O or NH; R⁴ represents hydrogen, C₁₋₄alkyl or pyrimidinyl; R⁵ represents C₁₋₄alkyl; Z¹ represents NH; Z² represents O; and X represents O.

Particularly interesting compounds are those interesting compounds wherein L represents C₁₋₄alkyl or a radical of formula -Alk-R³ (c-1), -Alk-Y-R⁴ (c-2) or -Alk-Z¹-C(=O)-Z²-R⁵ (c-3); Alk represents C₂₋₄alkanediyl; R³ represents 4-methoxyphenyl or a radical of formula 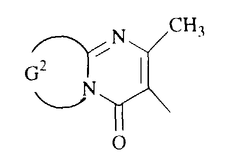 wherein G² represents CH=CH-CH=CH-, -S-(CH₂)₃-, -S-(CH₂)₂- or -S-CH=CH-; Y represents O or NH; and R⁴ represents C₁₋₄alkyl or 2-pyrimidinyl.

Other particularly interesting compounds are those interesting compounds wherein L represents hydrogen, C₁₋₄alkyloxy-carbonyl, phenylmethyl, hydroxyethyl or aminoethyl.

Especially interesting compounds are those particularly interesting compounds wherein L represents methyl or a radical of formula -Alk-R³ (c-1), Alk represents 1,2-ethanediyl and R³ represents 4-methoxyphenyl or a radical of formula 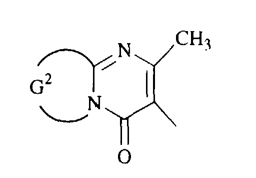 wherein G² represents -CH=CH-CH=CH-, -S-(CH₂)₃-, -S-(CH₂)₂- or -S-CH=CH-.

In order to simplify the structural representation of some of the compounds and intermediates in the following preparations the moiety containing the imidazole group fused to a benzene, pyridine or pyrimidine ring will hereinafter be represented by the symbol Q.

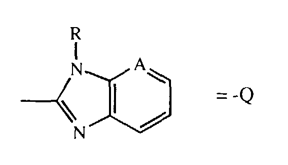

The compounds of formula (I) can generally be prepared by reacting an intermediate of formula (II) with an appropriately substituted diamine of formula (III).

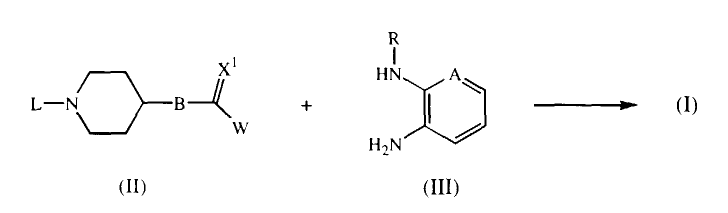

In this and the following reaction schemes W represents an appropriate reactive leaving group such as, for example, halo, e.g. chloro, bromo or iodo; C₁₋₆alkyloxy; C₁₋₆alkylthio, aryloxy or arylthio; and X¹ denotes O, S or NH.

The derivatives of formula (II) wherein B is CH₂ and W is halo may be generated in situ, for example, by halogenating the corresponding carboxylic acid with thionyl chloride, phosphorous trichloride, phosphoryl chloride and, polyphosphoric acid. The reaction of (II) with (III) may be conducted in a suitable reaction-inert solvent such as, for example, a hydrocarbon, e.g., benzene and, hexane; an ether, e.g., 1,1'-oxybisethane and tetrahydrofuran; a ketone, e.g., 2-propanone and, 2-butanone; an alcohol, e.g., methanol, ethanol, 2-propanol, and 1-butanol; a halogenated hydrocarbon, e.g., trichloromethane and, dichloromethane; an organic acid, e.g., acetic acid and, propanoic acid; a dipolar aprotic; solvent e.g., N,N-dimethylformamide, and N,N-dimethylacetamide; or a mixture of such solvents. Depending upon the nature of the solvent and W it may be appropriate to add to the reaction mixture a base such as is commonly employed in the art of conducting N-alkylation reactions and/or a iodide salt such as an alkali metal iodide. Elevated temperatures and stirring may enhance the reaction rate.

In some instances the reaction of (II) with (III) may first yield an intermediate of formula (II-a) which subsequently may be cyclized to the desired compound of formula (I), either in situ or, if desired, after isolation and purification.

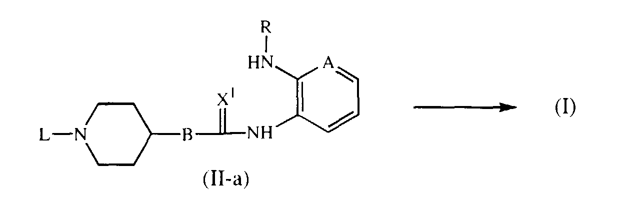

The compounds of formula (I) can also be prepared by reacting an intermediate of formula (IV) with an intermediate of formula (V) following art-known substitution reaction procedures. In (IV) and hereinafter, M is hydrogen when B is other than CH₂, or M represents an alkali or earth alkaline metal such as, for example, lithium or magnesium, when B represents CH₂.

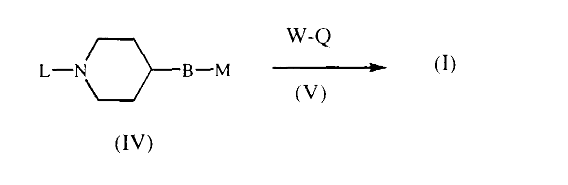

Similarly, the compounds of formula (I) can also be prepared by reacting an intermediate of formula (VI) with an intermediate of formula (VII) wherein M has the previously defined meaning. In formula (VI) and hereinafter W¹ represents an appropriate leaving group such as, for example, halo, e.g., chloro, and bromo; or a sulfonyloxy group such as, for example, methanesulfonyloxy and, 4-methylbenzenesulfonyloxy.

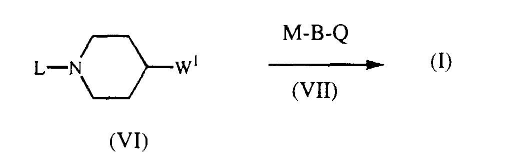

The compounds of formula (I) wherein B is -CH₂-, said compounds being represented by formula (I-a), can also be prepared by reacting an intermediate of formula (VIII) with an intermediate of formula (IX) or alternatively, by reacting an intermediate of formula (X) with an intermediate of formula (XI).

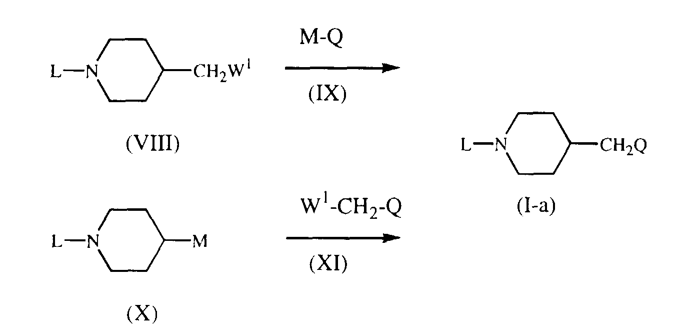

The reactions of (IV). (VI) , (VIII) and (X) with respectively (V), (VII), (IX) and (XI) may conveniently be conducted in an appropriate reaction-inert solvent such as for example, an aromatic hydrocarbon, e.g., benzene and methylbenzene; an ether, e.g. 1,4-dioxane, 1,1'-oxybisethane and tetrahydrofuran; a halogenated hydrocarbon, e.g. trichloromethane; N,N-dimethylformamide; N,N-dimethylacetamide; nitrobenzene; dimethylsulfoxide; 1-methyl-2-pyrrolidinone; and when M is hydrogen, said solvent may also be a C₁₋₆alkanol, e.g., methanol, ethanol, and 1-butanol; a ketone. e.g., 2-propanone and, 4-methyl-2-pentanone. In some instances, particularly when B is NH, the addition of an appropriate base such as, for example, an alkali metal carbonate or hydrogen carbonate, e.g., sodium carbonate, and sodium hydrogen carbonate; sodium hydride; or an organic base such as, for example, N,N-diethylethanamine or N-(1-methylethyl)-2-propanamine and/or the addition of an iodide salt, preferably an alkali metal iodide, may be appropriate. Somewhat elevated temperatures and stirring may enhance the rate of the reaction. A convenient alternative for reacting the intermediate of formula (IV) wherein -B-M represents -NH₂ with the reagents of formula (V) comprises stirring and heating the reactants in the presence of copper metal in a reaction-inert solvent such as described hereinbefore, in particular a dipolar aprotic solvent, e.g. N,N-dimethyl-formamide, and N,N-dimethylacetamide.

The compounds of formula (I) wherein B is -NH-, said compounds being represented by formula (I-b), can also be prepared by reacting an intermediate of formula (XII) with an intermediate of formula (VII) wherein B-M represents a radical -NH₂, said intermediate being represented by formula (VII-a), following art-known reductive N-alkylation procedures.

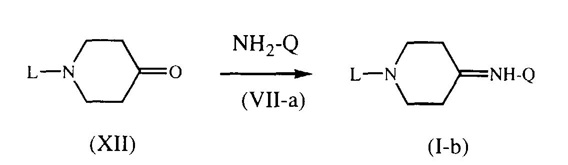

The reaction of (XII) with (VII-a) can conveniently be carried out by mixing the reactants in a suitable reaction-inert solvent with an appropriate reductant. Preferably, the ketone of formula (XII) is first reacted with the intermediate of formula (VII-a) to form an enamine, which optionally may be isolated and further purified, and subsequently reducing said enamine. Suitable solvents are, for example, water; C₁₋₆alkanols, e.g., methanol, ethanol, and 2-propanol ethers, e.g., 1,4-dioxane; halogenated hydrocarbons, e.g., trichloromethane; dipolar aprotic solvents, e.g., N,N-dimethylformamide, N,N-dimethylacetamide, and dimethylsulfoxide; or a mixture of such solvents. Appropriate reductants are for example, metal or complex metal hydrides, e.g., sodium borohydride, sodium cyanoborohydride, and lithium aluminum hydride. Alternatively, hydrogen in the presence of a suitable catalyst such as, for example, palladium-on-charcoal, and platinum-on-charcoal may be used as reductant. In order to prevent the undesired further hydrogenation of certain functional groups in the reactants and the reaction products it may be advantageous to add an appropriate catalyst poison to the reaction mixture such as, for example, thiophene.

The compounds of formula (I-b) can also be prepared by a cyclodesulfurization reaction of an appropriate thiourea of formula (II-a) wherein X¹ is S, said thiourea being represented by formula (II-a-1), which may be formed in situ by condensing an isothiocyanate of formula (XIII) with a diamine of formula (III).

Said cyclodesulfurization reaction may be carried out by reacting (II-a-1) with an appropriate alkyl halide, preferably iodomethane, in a suitable reaction-inert organic solvent such as a C₁₋₆alkanol, e.g., methanol, ethanol, and 2-propanol. Alternatively, said cyclodesulfurization reaction may also be carried out by the reaction of (II-a-1) with an appropriate metal oxide or salt such as, for example, a Hg(II) or Pb(II) oxide or salt, e.g., HgO, HgCl₂, Hg(OAc)₂, PbO or Pb(OAc)₂ in an appropriate solvent following art-known procedures. In some instances it may be appropriate to supplement the reaction mixture with a small amount of sulfur. Also methanediimides, especially dicyclohexylcarbodiimide may be used as cyclodesulfurizing agents.

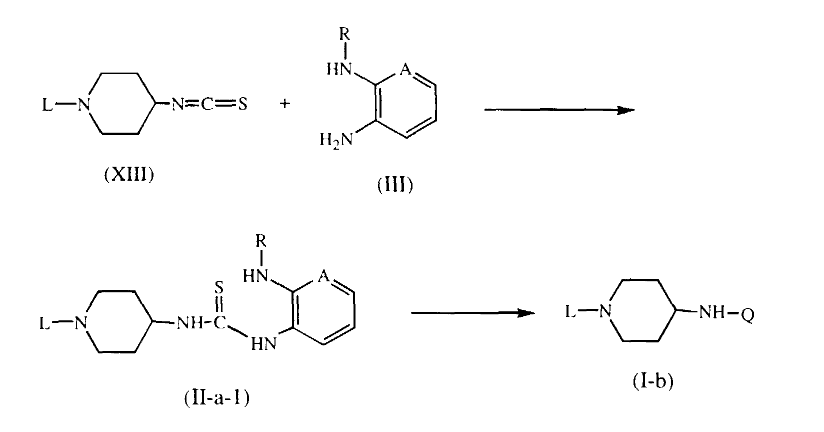

The compounds of formula (I) can also be prepared by N-alkylating an intermediate of formula (XV) with an appropriate alkylating reagent of formula (XIV).

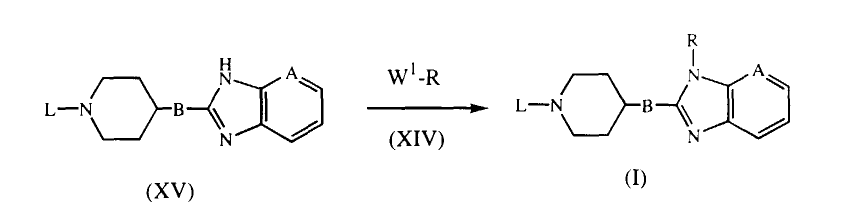

Said N-alkylation reaction can conveniently be conducted in a reaction-inert solvent such as, for example, water; an aromatic hydrocarbon, e.g., benzene, methylbenzene, and dimethylbenzene; an alkanol, e.g., methanol, ethanol, and 1-butanol; a ketone, e.g., 2-propanone, and 4-methyl-2-pentanone; an ether, e.g., tetrahydrofuran, 1,4-dioxane, and 1,1'-oxybisethane; a dipolar aprotic solvent, e.g., N,N-dimethylformamide, N,N-dimethylacetamide, dimethylsulfoxide, nitrobenzene and, 1-methyl-2-pyrrolidinone; or a mixture of such solvents. The addition of an appropriate base such as, for example, an alkali or an earth alkaline metal carbonate, hydrogen carbonate, alkoxide, hydride, amide, hydroxide or oxide, e.g., sodium carbonate, sodium hydrogen carbonate, potassium carbonate, sodium methoxide, sodium ethoxide, potassium tert. butoxide, sodium hydride, sodium amide, sodium hydroxide, calcium carbonate, calcium hydroxide, and calcium oxide; or an organic base, such as, for example, an amine, e.g., N,N-diethylethanamine, N-(1-methylethyl)-2-propanamine, 4-ethylmorpholine, and pyridine may be utilized to pick up the acid which is liberated during the course of the reaction. In some instances the addition of an iodide salt, preferably an alkali metal iodide, is appropriate. Somewhat elevated temperatures and stirring may enhance the rate of the reaction. Additionally, it may be advantageous to conduct said N-alkylation under an inert atmosphere such as, for example, oxygen-free argon or nitrogen.

Alternatively, said N-alkylation may be carried out by applying art-known conditions of phase transfer catalysis reactions. Said conditions comprise stirring the reactants with an appropriate base and optionally under an inert atmosphere as described hereinabove, in the presence of a suitable phase transfer catalyst such as, for example, a trialkylphenylmethylammonium, tetraalkylammonium. tetraalkylphosphonium, tetraarylphosphonium halide, hydroxide and, hydrogen sulfate.

The compounds of formula (I) wherein L is other than hydrogen, said L being represented by L¹, and said compounds being represented by formula (I-d) can also be prepared by N-alkylating a compound of formula (I) wherein L is hydrogen, said compound being represented by (I-e), with an alkylating reagent of formula (XVI).

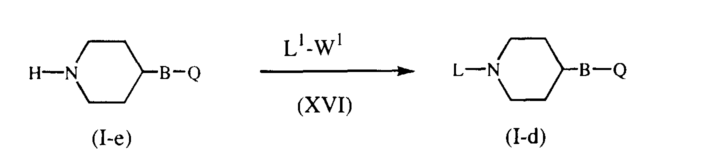

Said N-alkylation is conveniently conducted following art-known N-alkylation procedures as described hereinabove for the preparation of (I) from (XIV) and (XV).

The compounds of formula (I-d) wherein L is C₃₋₆cycloalkyl, C₁₋₁₂alkyl, a radical of formula (c-1), (c-2) or (c-3), said radicals being represented by the radical L²H- and said compounds by formula (I-d-1) can also be prepared by the reductive N-alkylation reaction of (I-e) with an appropriate ketone or aldehyde of formula L²=O (XVII), said L²=O being an intermediate of formula L²H₂ wherein two geminal hydrogen atoms are replaced by =O, and L² is a geminal bivalent radical comprising C₃₋₆cycloalkylidene, C₁₋₁₂alkylidene, R³-C₁₋₆alkylidene, R⁴-Y-C₁₋₆alkylidene and R⁵-Z²-C(=O)-Z¹-C₁₋₆alkylidene. 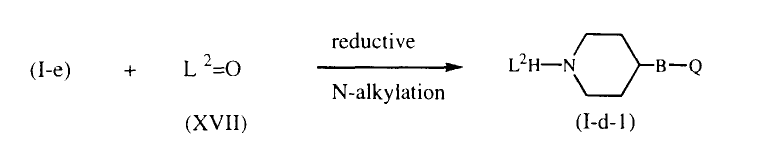 Said reductive N-alkylation can conveniently be carried out following the procedures described hereinabove for the preparation of compounds of formula (I-b) from (VII-a) and (XII), more particularly following the catalytic hydrogenation procedures.

The compounds of formula (I) wherein L is a radical of formula (c-2) and R⁴ is phenyl optionally substituted with halo, C₁₋₆alkyl, hydroxy or C₁₋₆alkyloxy or Het, said R⁴ being represented by R^{4-a} and said compounds by formula (I-d-2) may also be prepared by alkylating a compound of formula (I) wherein L is a radical of formula (c-2) and R⁴ is hydrogen, said compounds being represented by formula (I-d-3), with a reagent of formula (XVIII).

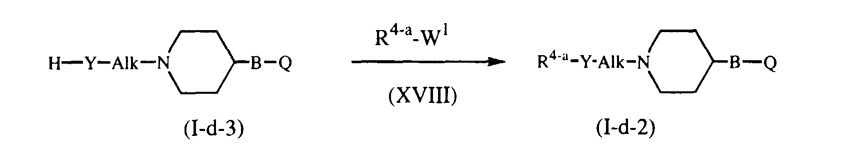

Similarly, the compounds of formula (I-d-2) may also be prepared by treating a compound of formula (I-d-4) with a reagent of formula (XIX).

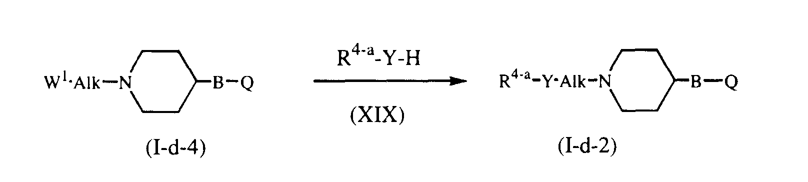

The alkylation reactions of (I-d-3) with (XVIII) and (I-d-4) with (XIX) may conveniently be conducted in an inert organic solvent such as, for example, an aromatic hydrocarbon, e.g., benzene, methylbenzene, dimethylbenzene; a ketone, e.g., 2-propanone, 4-methyl-2-pentanone; an ether, e.g., 1,4-dioxane, 1,1'-oxybisethane, tetrahydrofuran; and a dipolar aprotic solvent, e.g., N,N-dimethylformamide; N,N-dimethylacetamide; dimethyl sulfoxide; nitrobenzene; l-methyl-2-pyrrolidinone; and the like. The addition of an appropriate base such as, for example, an alkali metal carbonate or hydrogen carbonate, sodium hydride or an organic base such as, for example, N,N-diethylethanamine or N-(1-methylethyl)-2-propanamine may be utilized to pick up the acid which is liberated during the course of the reaction. Somewhat elevated temperatures may enhance the rate of the reaction.

The compounds of formula (I) wherein L is a radical of formula (c-3), Z¹ is NH, Z² being represented by Z^{2-a}, and said compounds by (I-d-5), can be prepared by reacting an isocyanate of formula (XXI) with a reagent of formula (XX).

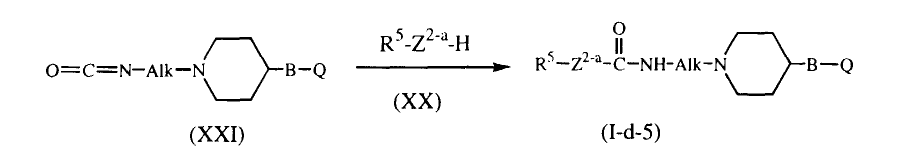

The compounds of formula (I) wherein L is a radical of formula (c-3), Z² is NH, Z¹ is other than a direct bond, said Z¹ being represented by Z^{1-a}, and said compounds by (I-d-6), can be prepared by reacting an isocyanate of formula (XXII) with a compound of formula (I-d-7).

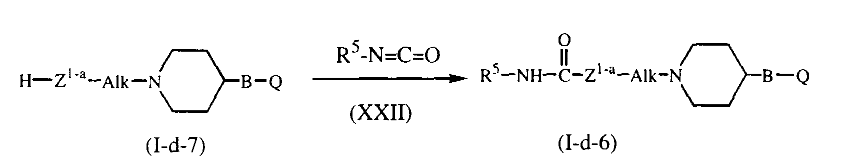

The reaction of (XX) with (XXI), or (XXII) with (I-d-7) can generally be conducted in a suitable reaction-inert solvent such as, for example, an ether, e.g., tetrahydrofuran, a halogenated hydrocarbon, e.g., trichloromethane. Elevated temperatures may be suitable to enhance the rate of the reaction.

The compounds of formula (I) wherein L is a radical of formula L³-C₂₋₆alkane-diyl, said L³ being phenyl optionally substituted with halo, C₁₋₆alkyl, hydroxy or C₁₋₆alkyloxy, Het or a radical of formula R⁵-Z²-C(=O)-, and said compounds being represented by formula (I-d-9), may also be prepared by the addition reaction of a compound of formula (I-e) to an appropriate alkene of formula (XXIV).

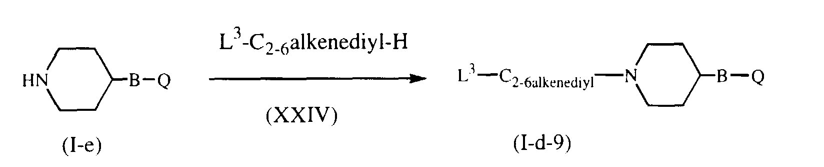

The compounds of formula (I) wherein L is 2-hydroxy-C₂₋₆alkyl, said compounds being represented by formula (I-d-10), can be prepared by reacting a compound of formula (I-e) with an epoxide (XXV) wherein R¹² is hydrogen, C₁₋₄alkyl or a radical R⁶-O-CH₂-.

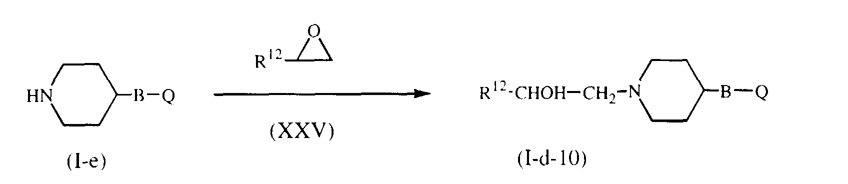

The reaction of (I-e) with respectively (XXIV) and (XXV) may be conducted by stirring and. if desired, heating the reactants in a reaction-inert solvent such as, for example, a ketone, e.g., 2-propanone, 4-methyl-2-pentanone, an ether, e.g., tetrahydrofuran, 1,1'-oxybisethane, an alcohol, e.g., methanol, ethanol, 1-butanol, a dipolar aprotic solvent, e.g., N,N-dimethylformamide and, N,N-dimethylacetamide.

The compounds of formula (I) wherein R³, R⁴ or R⁵ are Het, may also be prepared following art-known procedures for preparing heterocyclic ring systems or following analogous methods. A number of such cyclization procedures are described in for example, US-A-4,695,575 and in the references cited therein, in particular US-A-4,335,127; US-A-4,342,870 and US-A-4,443,451.

The compounds of formula (I) can also be converted into each other following art-known procedures of functional group transformation. Some examples of such procedures are cited hereinafter. The compounds of formula (I) containing a cyano substituent can be converted into the corresponding amines by stirring and, if desired, heating the starting cyano compounds in a hydrogen containing medium in the presence of an appropriate catalyst such as, for example, platinum-on-charcoal and Raney-nickel. Suitable solvents are, for example, methanol and, ethanol. Amino groups may be alkylated or acylated following art-known procedures such as, for example, N-alkylation, N-acylation, and reductive N-alkylation. The compounds of formula (I) containing an amino group substituted with a radical arylmethyl, may be hydrogenolyzed by treating the starting compound with hydrogen in the presence of a suitable catalyst, e.g., palladium-on-charcoal, and platinum-on-charcoal, preferably in an alcoholic medium. The compounds of formula (I) wherein L is methyl or phenylmethyl can be converted into compounds of formula (I) wherein L is a C₁₋₆alkyloxycarbonyl group by reacting the methyl or phenylmethyl derivative with C₁₋₆alkyloxycarbonyl halides such as, for example, ethyl chloroformate in a suitable reaction-inert solvent and in the presence of a base like N,N-diethylethanamine. The compounds of formula (I) wherein L is hydrogen can be obtained from compounds of formula (I) wherein L is phenylmethyl or C₁₋₆alkyloxycarbonyl following art-know procedures like catalytic hydrogenation or hydrolysis in an acidic or alkaline medium depending on the nature of L.

In all of the foregoing and in the following preparations, the reaction products may be isolated from the reaction mixture and, if necessary, further purified according to methodologies generally known in the art.

Some intermediates and starting materials in the foregoing preparations are known compounds which may be prepared according to art-known methodologies of preparing said or similar compounds and others are new. A number of such preparation methods will be described hereinafter in more detail.

Starting materials such as the intermediates of formulae (II), (IV), (VI), (VIII), (X), (XII), (XIII) and (XV) can conveniently be prepared following procedures similar to those described in for example, US-A-4,219,559; US-A-4,556,660; US-A-4,634,704; US-A-4,695,569; US-A-4,695,575, US-A-4,588,722, US-A-4,835,161 and US-A-4,897,401 and in EP-A-0,206,415; EP-A-0,282,133; EP-A-0,297,661 and EP-A-0,307,014.

The intermediates of formula (III) can be prepared from an aromatic starting material with vicinal halo and nitro substituents (XXVII) by reaction with a suitable amine of formula (XXVI), followed by art-known nitro-to-amine reduction.

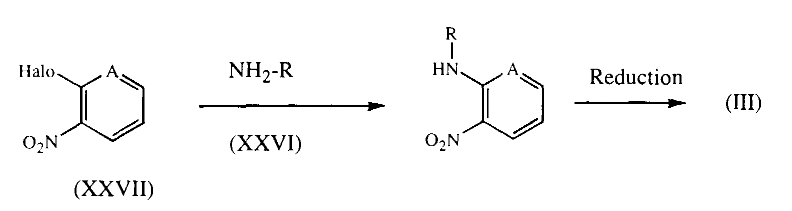

The intermediates of formulae (V), (VII), (IX) and (XI) then, can be prepared from the intermediates of formula (III) following art-known procedures of converting aromatic products with vicinal amino groups into benzimidazoles, imidazopyridines and/or purines.

The compounds of formula (I), the pharmaceutically acceptable acid addition salts and stereochemically isomeric forms thereof possess useful pharmacological properties. More particularly, they are active antiallergic and antihistaminic compounds which activity can be demonstrated by, e.g., the results obtained in the test "Protection of Rats from Compound 48/80-induced lethality", the "PCA (passive cutane anaphylaxis)-test in Rats" described in Drug Dev. Res., 5, 137-145 (1985), the "Histamine-induced lethality test in Guinea Pigs" and the "Ascaris Allergy test in Dogs". The latter two tests are described in Arch. Int. Pharmacodyn. Ther. 251, 39-51 (1981).

An interesting feature of the present compounds resides in their rapid onset of action and favorable duration of action.

In view of their antiallergic properties, the compounds of formula (I) and their acid addition salts are very useful in the treatment of broad range of allergic diseases such as, for example, allergic rhinitis, allergic conjunctivitis, chronic urticaria, allergic asthma and the like.

In view of their useful antiallergic properties the subject compounds may be formulated into various pharmaceutical forms for administration purposes. To prepare the antiallergic compositions of this invention, an effective amount of the particular compound, in base or acid addition salt form, as the active ingredient is combined in intimate admixture with a pharmaceutically acceptable carrier, which carrier may take a wide variety of forms depending on the form of preparation desired for administration. These pharmaceutical compositions are desirably in unitary dosage form suitable, preferably, for administration orally, rectally, percutaneously, or by parenteral injection. For example, in preparing the compositions in oral dosage form, any of the usual pharmaceutical media may be employed such as, for example, water, glycols, oils, and alcohols in the case of oral liquid preparations such as suspensions, syrups, elixirs and solutions: or solid carriers such as starches, sugars, kaolin, lubricants, binders, and disintegrating agents in the case of powders, pills, capsules and tablets. Because of their ease in administration, tablets and capsules represent the most advantageous oral dosage unit form, in which case solid pharmaceutical carriers are obviously employed. For parenteral compositions, the carrier will usually comprise sterile water, at least in large part, though other ingredients, for example to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. In the compositions suitable for percutaneous administration, the carrier optionally comprises a penetration enhancing agent and/or a suitable wetting agent, optionally combined with suitable additives of any nature in minor proportions, which additives do not introduce a significant deleterious effect on the skin. Said additives may facilitate the administration to the skin and/or may be helpful for preparing the desired compositions. These compositions may be administered in various ways, e.g., as a transdermal patch, as a spot-on or as an ointment. Acid addition salts of (I) due to their increased water solubility over the corresponding base form, are obviously more suitable in the preparation of aqueous compositions.

It is especially advantageous to formulate the aforementioned pharmaceutical compositions in dosage unit form for ease of administration and uniformity of dosage. Dosage unit form as used in the specification and claims herein refers to physically discrete units suitable as unitary dosages, each unit containing a predetermined quantity of active ingredient calculated to produce the desired therapeutic effect in association with the required pharmaceutical carrier. Examples of such dosage unit forms are tablets (including scored or coated tablets), capsules, pills, powder packets, wafers, injectable solutions or suspensions, teaspoonfuls, tablespoonfuls and the like, and segregated multiples thereof.

The present invention also relates to a method of treating warm-blooded animals suffering from said allergic diseases by administering to said warm-blooded animals an effective antiallergic amount of a compound of formula (I) or a pharmaceutically acceptable acid addition salt form thereof.

Those of skill in treating allergic diseases in warm-blooded animals could easily determine the effective amount from the test results presented hereinafter. In general it is contemplated that an effective antiallergic amount would be from 0.001 mg/kg to 20 mg/kg body weight, and more preferably from 0.01 mg/kg to 5 mg/kg body weight.

The following examples are intended to illustrate the present invention in all its aspects. Unless otherwise stated all parts therein are by weight.

### Experimental part

### A. Preparation of the intermediates

### Example 1

To a mixture of 15.5 parts of 2-chloro- 1H-benzimidazole and 235 parts of N,N-dimethylacetamide there were added 22 parts of 4-(chloromethyl)-2-methylthiazole monohydrochloride and 25.4 parts of sodium carbonate. The whole was stirred for 18 hours at 75°C and was then poured into water. The product was extracted with 4-methyl-2-pentanone and the extract was washed with water, dried, filtered and evaporated. The residue was crystallized from 2,2'-oxybispropane, yielding 11.3 parts (42.8%) of 2-chloro-1-[(2-methyl-4-thiazolyl)methyl]-1H-benzimidazole (interm. 1). In a similar manner there was also prepared 2-chloro-1-[(6-methyl-2-pyridinyl)methyl]-1H-benzimidazole (interm. 2).

### Example 2

a) To a stirred and refluxing mixture of 60 parts of 4-fluorobenzenethiol, 93 parts of 1-bromo-3-chloropropane, 100 parts of ethanol and 45 parts of water there was added dropwise a solution of 19 parts of sodium hydroxide in 80 parts of water. Stirring at reflux temperature was continued for 8 hours. After cooling, the organic layer was separated and distilled under reduced pressure (1.7 10³ Pa), yielding two fractions of resp. 53 parts (bp. 136-140°C) and 32 parts (bp. 140-152°C) of product. Total yield : 85 parts of 1-[(3-chloropropyl)thio]-4-fluorobenzene (interm. 3).
b) A mixture of 67.5 parts of intermediate 3; 42.9 parts of 1,4-dioxa-8-azaspiro[4,5]-decane, 47.7 parts of sodium carbonate, a few crystals of potassium iodide and 2400 parts of 4-methyl-2-pentanone was stirred for 70 hours at reflux temperature. The reaction mixture was filtered while hot and the filtrate was washed with 1,1'-oxybisethane and evaporated. The residue was triturated in 2,2'-oxybispropane while cooling at -20°C. A first fraction of 4.4 parts of product was obtained by filtration. Evaporation of the mother liquor yielded a second fraction of 97 parts of product. Total yield : 101.4 parts of 1,4-dioxa-8-[3-[(4-fluorophenyl)thio]propyl]-8-azaspiro[4,5]decane; mp. 135.5-140°C (interm. 4)

### Example 3

a) A mixture of 2.44 parts of 6-methyl-2-pyridinemethanamine, 3.2 parts of 2-chloro-3-nitropyridine, 1.7 parts of sodium hydrogen carbonate and 120 parts of ethanol was stirred for 3 hours at reflux temperature. The reaction mixture was filtered while hot over diatomaceous earth. After cooling, the precipitate which formed in the filtrate was filtered off and dried, yielding 2.5 parts (51%) of 6-methyl-N-(3-nitro-2-pyridinyl)-2-pyridinemethanamine; mp. 131.7°C (interm. 5).
b) A mixture of 55 parts of intermediate 5; 2 parts of a solution of thiophene in methanol 4% and 480 pans of methanol was hydrogenated at normal pressure and at 50°C with 3 parts of platinum-on-charcoal catalyst 5%. After the calculated amount of hydrogen was taken up, the catalyst was filtered off over diatomaceous earth and the filtrate was evaporated, yielding 47 parts (99.7%) of N²-[(6-methyl-2-pyridinyl)-methyl]-2,3-pyridinediamine (interm. 6).
c) A mixture of 47 parts of ethyl 4-isothiocyanato-1-piperidinecarboxylate, 47 parts of intermediate 6 and 900 parts of tetrahydrofuran was stirred overnight at room temperature. There was added 2,2'-oxybispropane to enhance crystallization. The product was filtered off and dried, yielding 78.5 parts (83.5%) of ethyl 4-[[[[2-[[(6-methyl-2-pyridinyl)methyl]amino]-3-pyridinyl]amino]thioxomethyl]amino]-1-piperidine-carboxylate; mp. 176°C (interm. 7).

### B. Preparation of the final compounds

### Example 4

To a stirred mixture of 45 parts of 2-[[1-(phenylmethyl)-4-piperidinyl]methyl]-1H-benzimidazole and 376 parts of N,N-dimethylformamide there were added portion wise 12.2 parts of a dispersion of sodium hydride in mineral oil (60%) and, after stirring for 1/2 hour, a solution of 28 parts of 2-(chloromethyl)-6-methylpyridine monohydrochloride in some N,N-dimethylformamide. Stirring at room temperature was continued overnight. After the addition of ethanol, the reaction mixture was poured into water. The product was extracted with methylbenzene and the extract was dried, filtered and evaporated. The residue was purified by column chromatography (silica gel ; CH₂Cl₂/ CH₃OH 95:5). The eluent of the desired fraction was evaporated and the residue was converted into the ethanedioate (1:5/2) salt in acetonitrile. The product was filtered off and dried, yielding 27.8 parts (29.2%) of 1-[(6-methyl-2-pyridinyl)methyl]-2-[[1-(phenylmethyl)-4-piperidinyl]methyl]-1H-benzimidazole ethanedioate (1:5/2); mp. 155.3°C (comp. 48).

### Example 5

To a stirred mixture of 36.7 parts of compound 48 and 267 parts of tetrahydrofuran there were added 15.68 parts of ethyl chloroformate. Stirring was continued for 6 hours and then there were added 11.7 parts of N,N-diethylethanamine. After stirring over weekend, the reaction mixture was evaporated and the residue was taken up in water. The product was extracted with dichloromethane and the extract was dried, filtered and evaporated. The residue was purified by column chromatography (silica gel ; CH₂Cl₂/ CH₃OH 95:5). The eluent of the desired fraction was evaporated and the residue was crystallized from acetonitrile. The product was filtered off and dried, yielding 15.65 parts (44.3%) of ethyl 4-[[1-[(6-methyl-2-pyridinyl)methyl-1H-benzimidazol-2-yl]-methyl]-1-piperidinecarboxylate; mp. 159.7°C (comp. 53).

### Example 6

A mixture of 2.9 parts of ethyl 4-[(1H-benzimidazol-2-yl)amino]-1-piperidinecarboxylate, 1.8 parts of 4-(chloromethyl)-2-methylthiazole monohydrochloride, 2.12 parts of sodium carbonate and 45 parts of N,N-dimethylacetamide was stirred overnight at 70°C. The reaction mixture was poured into water and the product was extracted with 4-methyl-2-pentanone. The extract was dried, filtered and evaporated and the residue was crystallized from acetonitrile. The product was filtered off and dried, yielding 1.5 parts (37.5%) of ethyl 4-[[1-[(2-methyl-4-thiazolyl)methyl]-1H-benzimidazol-2-yl]-amino]-1-piperidinecarboxylate; mp. 160°C (comp. 12).

### Example 7

A mixture of 25 parts of compound 12; 34 parts of potassium hydroxide and 160 parts of 2-propanol was stirred overnight at reflux temperature. The reaction mixture was evaporated and the residue was taken up in water. The product was extracted with dichloromethane and the extract was dried, filtered and evaporated. The residue was converted into the trihydrochloride salt in 2-propanol. The product was filtered off and dried, yielding 20 parts (71.2%) of 1-[(2-methyl-4-thiazolyl)methyl]-N-(4-piperidinyl)-1H-benzimidazol-2-amine trihydrochloride hemihydrate; mp. 206.4°C (comp. 13).

### Example 8

A mixture of 15 parts of compound 58 and 224 parts of hydrobromic acid 48% was refluxed for 3 hours. The reaction mixture was evaporated and the residue was taken up in water. After basifying with sodium hydroxide solution, the product was extracted with dichloromethane. The extract was dried, filtered and evaporated. The residue was crystallized from acetonitrile, yielding 5 parts (41.3%) of 1-[(4-methyl-2-thiazolyl)-methyl]-N-(4-piperidinyl)-1H-benzimidazol-2-amine (comp. 59).

### Example 9

A mixture of 2.3 parts of 6-(2-chloroethyl)-7-methyl-5H-thiazolo[3,2-a]pyrimidin-5-one, 3.3 parts of compound 13; 1.6 parts of sodium carbonate and 160 parts of 4-methyl-2-pentanone was stirred for 48 hours at reflux temperature. The reaction mixture was evaporated and the residue was taken up in water. The product was extracted with dichloromethane and the extract was dried, filtered and evaporated. The residue was purified by column chromatography (silica gel ; CH₂Cl₂ / CH₃OH(NH₃) 95:5). The eluent of the desired fraction was evaporated and the residue was crystallized from acetonitrile. The product was filtered off and dried, yielding 1.64 parts (31.6%) of 7-methyl-6-[2-[4-[[1-[(2-methyl-4-thiazolyl)methyl]-1H-benzimidazol-2-yl]amino]-1-piperidinyl]ethyl]-5H-thiazolo[3,2-a]pyrimidin-5-one; mp. 129.8°C (comp. 18).

### Example 10

A mixture of 2.26 parts of 3-(2-chloroethyl)-2-methyl-4H-pyrido[1,2-a]pyrimidin-4-one, 3.2 parts of compound 2; 1.06 parts of sodium carbonate and 45 parts of N,N-dimethylacetamide was stirred overnight at 70°C. The reaction mixture was poured into water. The precipitate was filtered off and boiled in methanol. The product was filtered off while hot, yielding 2.1 parts (41.5%) of 2-methyl-3-[2-[4-[[3-[(6-methyl-2-pyridinyl)methyl]-3H-imidazo[4,5-b]pyridin-2-yl]amino]-1-piperidinyl]ethyl]-4H-pyrido[1,2-a]pyrimidin-4-one; mp. 233.1°C (comp. 7).

### Example 11

The following reaction was carried out under a nitrogen atmosphere. To a mixture of 7.5 parts of ethyl 4-hydroxy- 1 -piperidinecarboxylate and 94 parts of N,N-dimethylformamide there were added portionwise 2.1 parts of a dispersion of sodium hydride in mineral oil (50%). After stirring for 1 hour at room temperature and for 20 min at 40°C, there was added dropwise a solution of 11.3 parts of intermediate 1 in 94 parts of N,N-dimethylformamide. Stirring was continued overnight at room temperature. After addition of some ethanol, the reaction mixture was evaporated. The residue was poured into ice-water and the whole was extracted with dichloromethane. The extract was dried, filtered and evaporated. The residue was purified by column chromatography (silica gel ; CH₂Cl₂ / CH₃OH 90:10). The eluent of the desired fraction was evaporated, yielding 13 parts (75.5%) of ethyl 4-[[1-[(2-methyl-4-thiazolyl)methyl]-1H-benzimidazol-2-yl]-oxy]-1-piperidinecarboxylate (comp. 20).

### Example 12

A mixture of 4.5 parts of compound 13; 2 parts of polyoxymethylene, 5 parts of potassium acetate and 120 parts of methanol was hydrogenated at normal pressure and at 50°C with 1 part of palladium-on-charcoal catalyst 10%. After the calculated amount of hydrogen was taken up, the catalyst was filtered off over diatomaceous earth. The filtrate was evaporated and the residue was taken up in water. After basifying with sodium carbonate, the product was extracted with trichloromethane. The extract was dried, filtered and evaporated and the residue was converted into the ethanedioate (1:2) salt in methanol. The product was filtered off and dried, yielding 3.7 parts (70.9%) of N-(1-methyl-4-piperidinyl)-1-[(2-methyl-4-thiazolyl)methyl]-1H-benzimidazol-2-amine ethanedioate (1:2); mp. 221.3°C (comp. 16).

### Example 13

A mixture of 78 parts of intermediate 7; 58.5 parts of mercury(II)oxide, 1 part of sulfur and 800 parts of ethanol was stirred for 2 hours at reflux temperature. The reaction mixture was filtered over diatomaceous earth and the filtrate was evaporated, yielding 63.5 parts (88.5%) of ethyl 4-[[3-[(6-methyl-2-pyridinyl)methyl]-3H-imidazo[4,5-b]-pyridin-2-yl]amino]-1-piperidinecarboxylate (comp. 1).

### Example 14

Trough a stirred mixture of 3.23 parts of compound 2 and 80 parts of methanol there were bubled 0.9 parts of oxirane. Stirring was continued overnight at room temperature. The reaction mixture was evaporated and the residue was purified by column chromatography (silica gel ; CHCl₃ / CH₃OH 96:4). The eluent of the desired fraction was evaporated and the residue was crystallized from acetonitrile. The product was filtered off and dried, yielding 0.7 parts (19%) of 4-[[3-[(6-methyl-2-pyridinyl)methyl]-3H-imidazo[4,5-b]-pyridin-2-yl]amino]-1-piperidineethanol; mp. 152.0°C (comp. 4).

### Example 15

A mixture of 9 parts of compound 5; 11 parts of potassium hydroxide and 120 parts of 2-propanol was stirred for 4 hours at reflux temperature. The reaction mixture was evaporated and the residue was taken up in water. The product was extracted with dichloromethane (2x) and the combined extracts were dried, filtered and evaporated, yielding 7.5 parts (100%) of N-[1-(2-aminoethyl)-4-piperidinyl]-3-[(6-methyl-2-pyridinyl)methyl]-3H-imidazo[4,5-b]pyridin-2-amine (comp. 8).

### Example 16

A mixture of 1.2 parts of 2-chloropyrimidine, 3.7 parts of compound 8; 0.9 parts of sodium hydrogen carbonate and 80 parts of ethanol was stirred overnight at reflux temperature. The reaction mixture was filtered over diatomaceous earth and the filtrate was evaporated. The residue was crystallized from a mixture of acetonitrile and methanol. The product was filtered off and dried in vacuo at 130°C overnight, yielding I part (22.5%) of 3-[(6-methyl-2-pyridinyl)methyl]-N-[1-[2-[(2-pyrimidinyl)amino]-ethyl]-4-piperidinyl]-3H-imidazol[4,5-b]pyridin-2-amine; mp. 187.4°C (comp. 9).

All compounds listed in tables 1, 2, 3, 4 and 5 were prepared following methods of preparation described in examples 4 - 16, as is indicated in column 2 (Ex. No.)

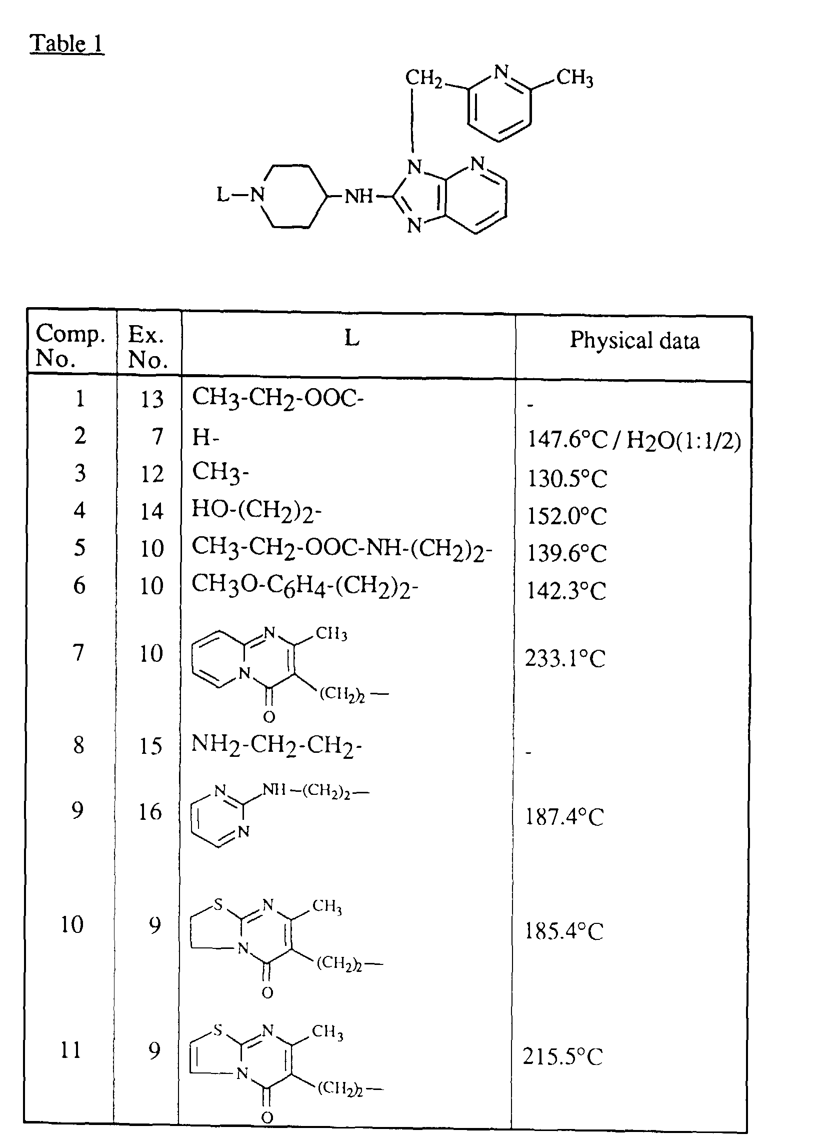

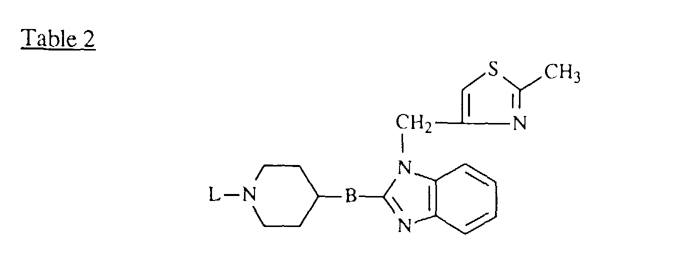

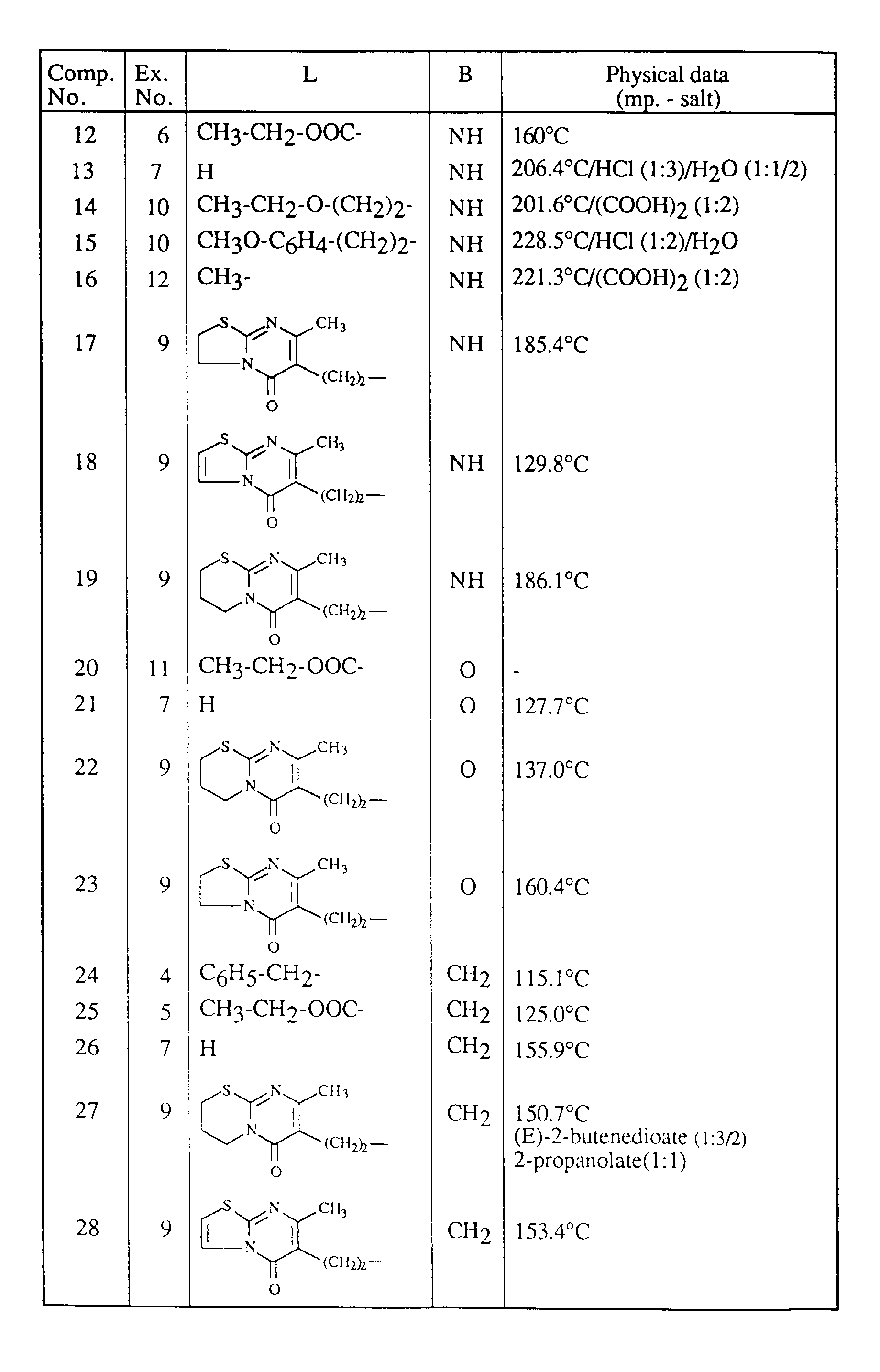

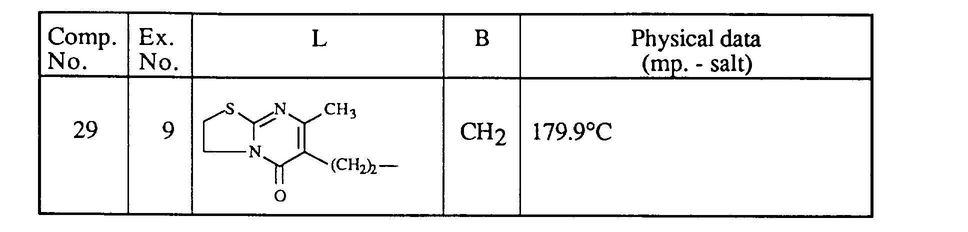

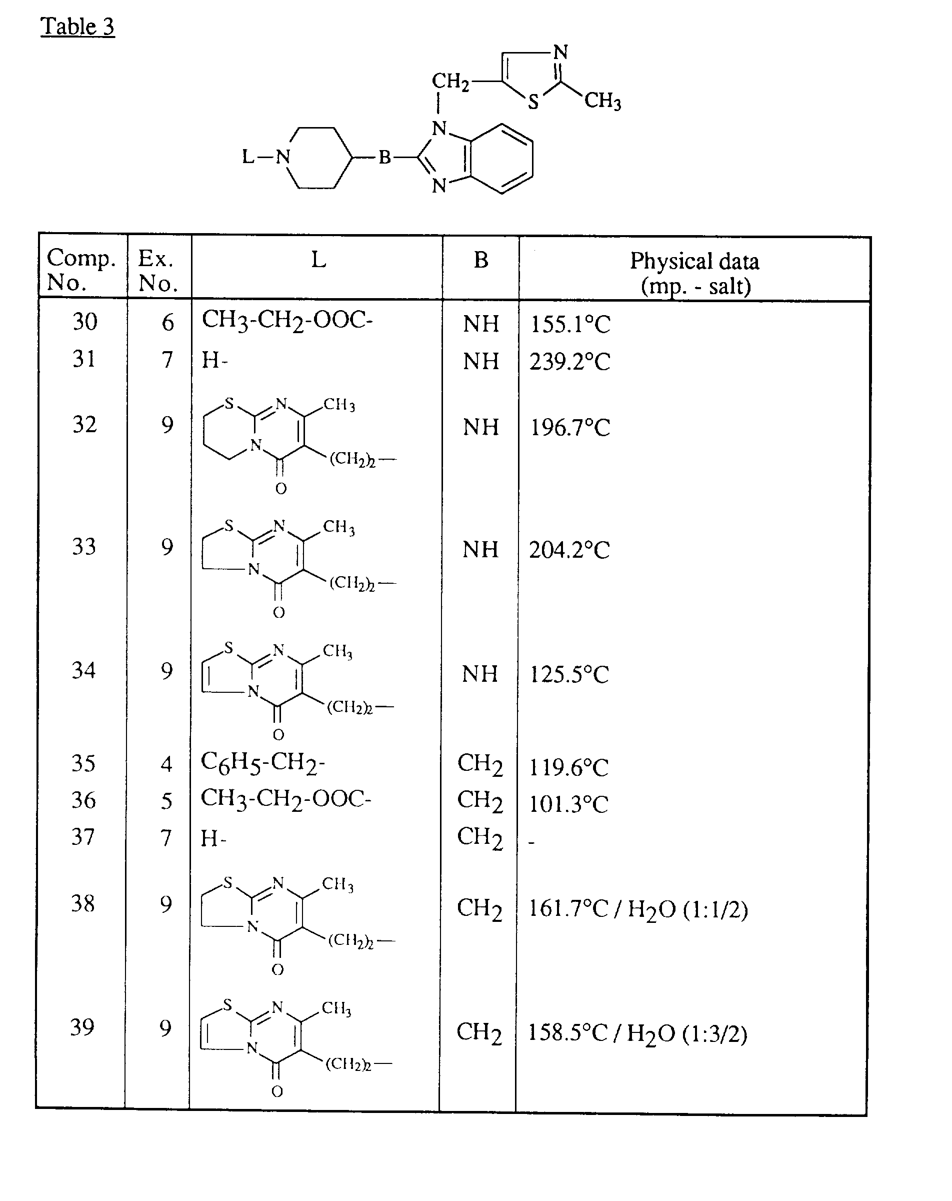

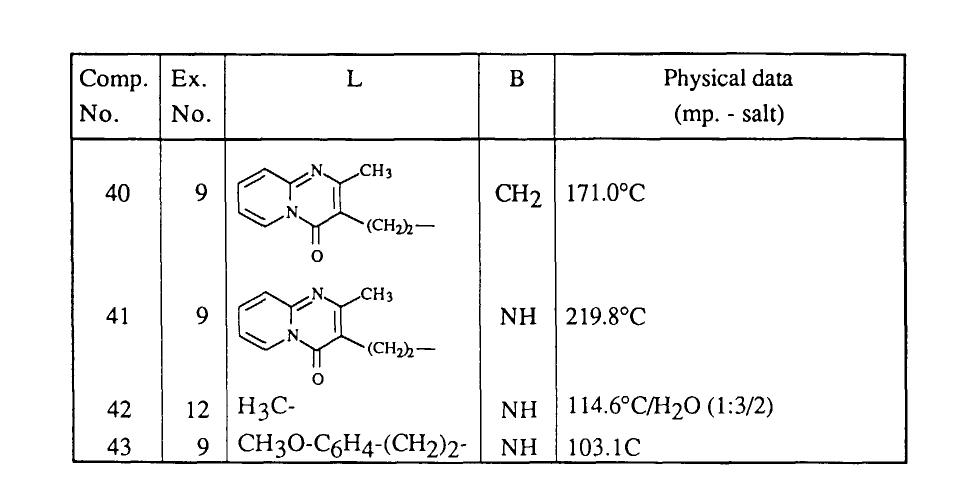

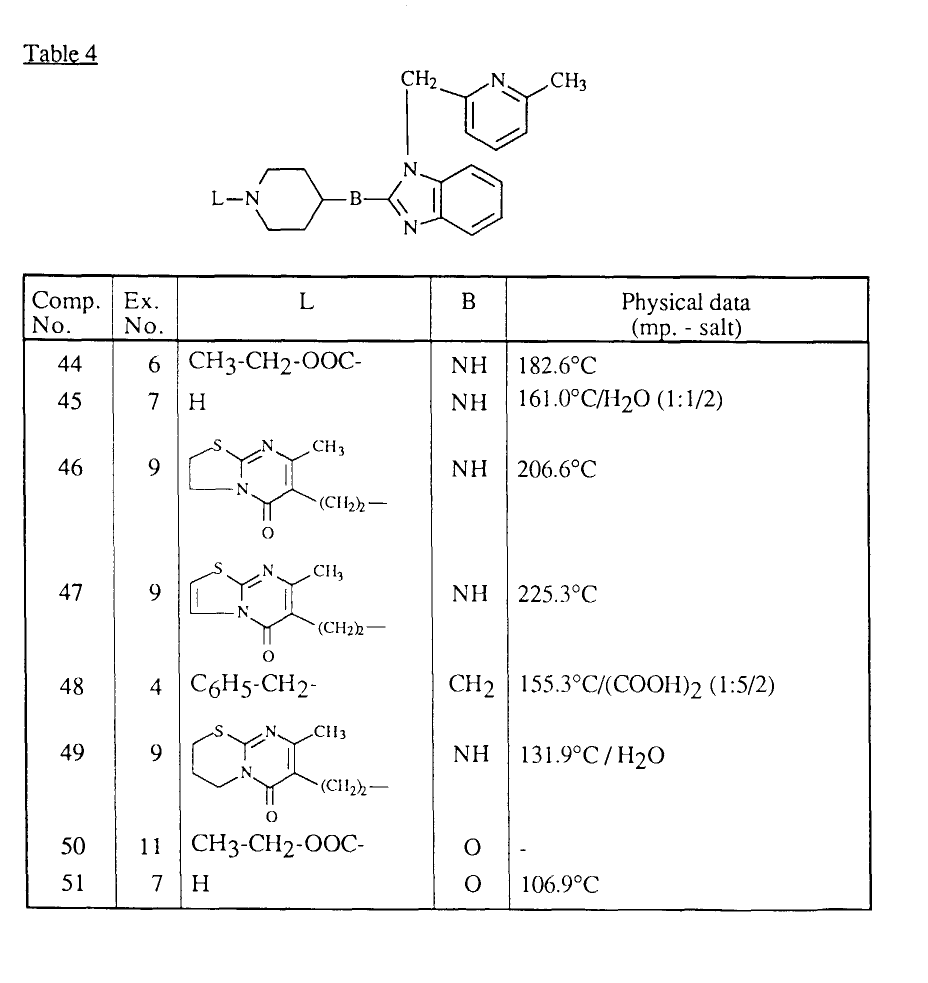

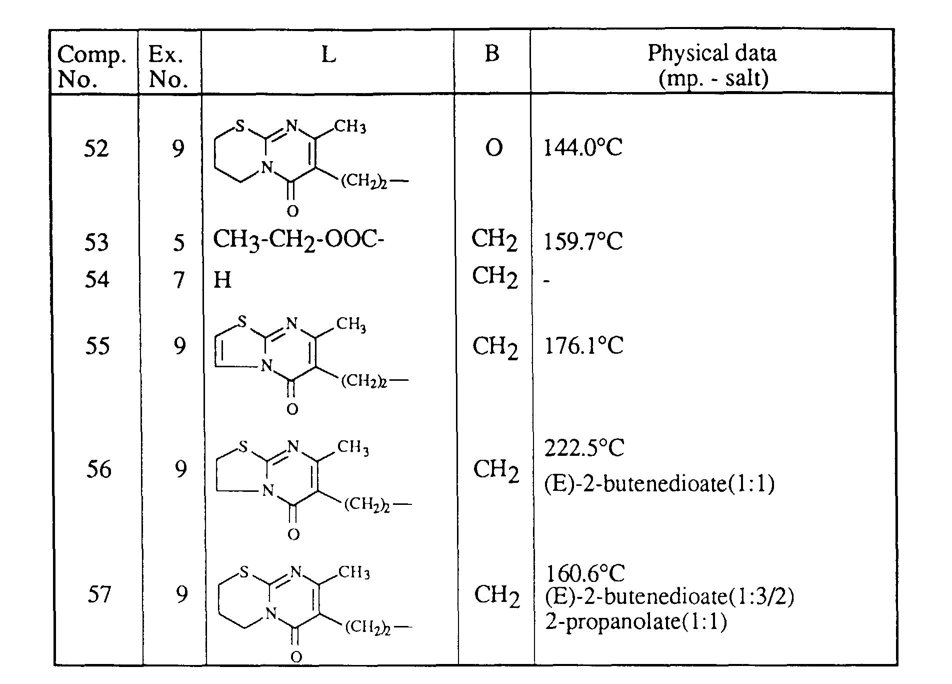

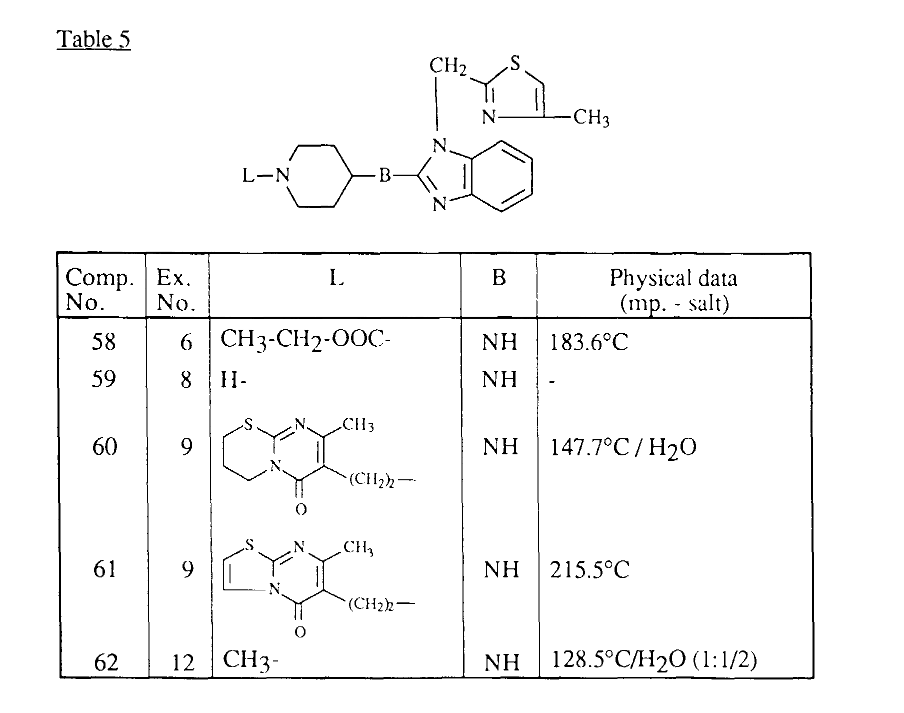

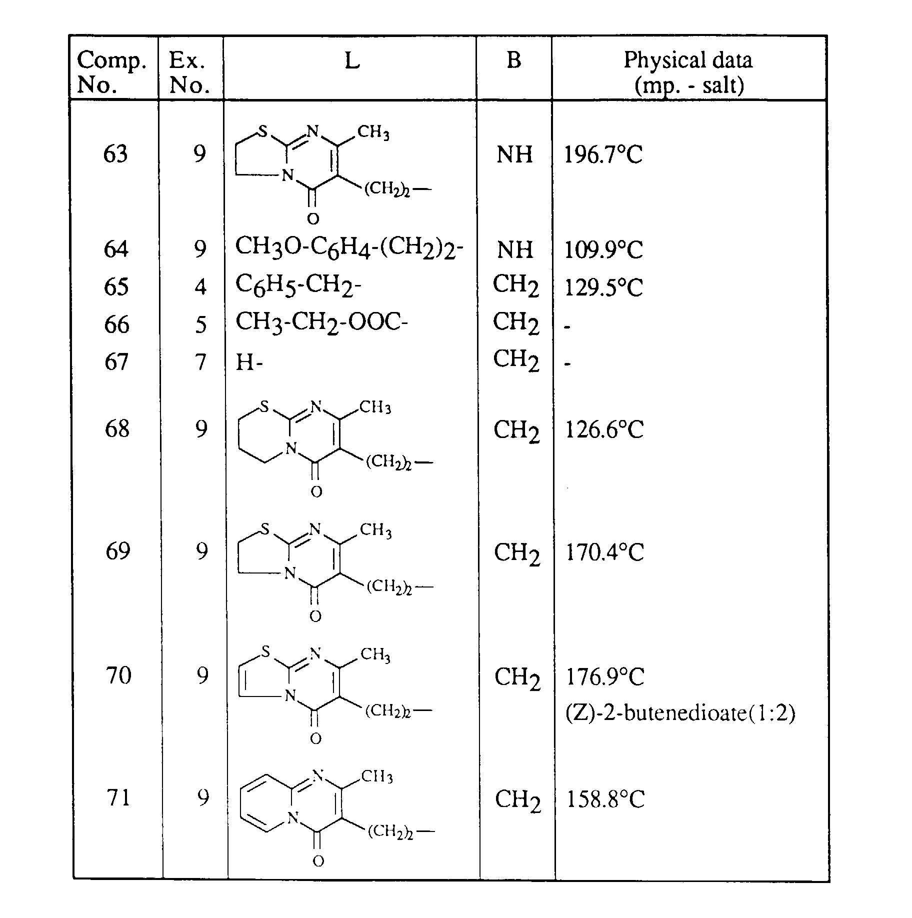

### C. Pharmacological example

### Example 17

The useful anti-allergic and anti-histaminic properties of the compounds of formula (I) can be demonstrated, e.g., in the test "Protection of rats from compound 48/80 - induced lethality" which is described in US-A-4,556,660. The compounds of formula (I) were administered subcutaneously and/or orally to rats. The ED₅₀-value (mg/kg) for the compounds 9, 16, 19, 23, 27, 28, 29, 55, 62, 69 and 70 was found to be 0.04 mg/kg.

## Claims

1. A compound having the formula : 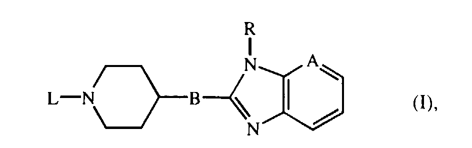 a pharmaceutically acceptable acid addition salt or a stereochemically isomeric form thereof, wherein
-A= is -N= or -CH=;
B represents NH or CH₂;
R is a radical of formula; 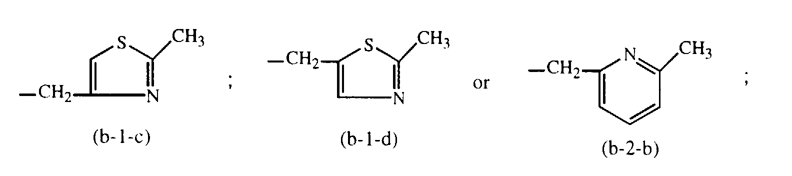
L is hydrogen; C₁₋₁₂alkyl; C₁₋₆alkyloxycarbonyl; or a radical of formula
-Alk-R³ (c-1);
-Alk-Y-R⁴ (c-2);
or
-Alk-Z¹-C(=O)-Z²-R⁵ (c-3);
wherein
R³ is Het or phenyl optionally substituted with halo, C₁₋₆alkyl, hydroxy or C₁₋₆alkyloxy;
R⁴ is hydrogen, C₁₋₆alkyl, Het or phenyl optionally substituted with halo, C₁₋₆alkyl, hydroxy or C₁₋₆alkyloxy;
R⁵ is hydrogen, C₁₋₆alkyl, Het or phenyl optionally substituted with halo, C₁₋₆alkyl, hydroxy or C₁₋₆alkyloxy;
Y is O or NH;
Z¹ and Z² each independently are O or NH;
each Alk independently is C₁₋₆alkanediyl;
Het is pyridinyl, optionally substituted with one or two substituents each independently selected from halo, amino, mono- and di(C₁₋₆alkyl)amino, nitro, cyano, aminocarbonyl, C₁₋₆alkyl, C₁₋₆alkyloxy, C₁₋₆alkylthio, C₁₋₆alkyloxycarbonyl, hydroxy, C₁₋₆alkylcarbonyloxy and carboxyl; pyridinyloxide, optionally substituted with nitro; pyrimidinyl, optionally substituted with one or two substituents each independently selected from halo, amino, C₁₋₆alkylamino, hydroxy, C₁₋₆alkyl, C₁₋₆alkyloxy and
C₁₋₆alkylthio; pyridazinyl, optionally substituted with C₁₋₆alkyl or halo; pyrazinyl, optionally substituted with halo, amino or C₁₋₆alkyl; thienyl, optionally substituted with halo or C₁₋₆alkyl; furanyl, optionally substituted with halo or C₁₋₆alkyl; pyrrolyl, optionally substituted with C₁₋₆alkyl; thiazolyl, optionally substituted with C₁₋₆alkyl or C₁₋₆alkyloxycarbonyl; imidazolyl, optionally substituted with one or two substituents each independently selected from C₁₋₆alkyl and nitro; tetrazolyl, optionally substituted with C₁₋₆alkyl; 1,3,4-thiadiazolyl, optionally substituted with C₁₋₆alkyl or amino; 5,6-dihydro-4H-1,3-thiazin-2-yl, optionally substituted with C₁₋₆alkyl; 4,5-di-hydrothiazolyl, optionally substituted with C₁₋₆alkyl; oxazolyl, optionally substituted with C₁₋₆alkyl; 4,5-dihydro-5-oxo-1H-tetrazolyl, optionally substituted with C₁₋₆alkyl; 1,4-dihydro-2,4-dioxo-3(2H)-pyrimidinyl, optionally substituted with C₁₋₆alkyl; 3,4-dihydro-4-oxopyrimidinyl or 4,5-dihydro-4-oxopyrimidinyl, both radicals optionally substituted with up to 3 substituents selected from C₁₋₆alkyl, amino, C₁₋₆alkylaminocarbonylamino, arylaminocarbonylamino, arylC₁₋₆alkylamino and C₁₋₆alkylamino; 2,3-dihydro-3-oxopyridazinyl; 2-oxo-3-oxazolidinyl; pyrrolidinyl; piperidinyl; morpholinyl; thiomorpholinyl; dioxanyl, optionally substituted with C₁₋₆alkyl; indolyl, optionally substituted with hydroxy or C₁₋₆alkyl; quinolinyl, optionally substituted with hydroxy or C₁₋₆alkyl; quinazolinyl, optionally substituted with hydroxy or C₁₋₆alkyl; quinoxalinyl, optionally substituted with C₁₋₆alkyl; phthala-zinyl, optionally substituted with halo; 1,3-dioxo-1H-isoindol-2(3H)-yl; 2,3-dihydro-3-oxo-4H-benzoxazinyl and 2,3-dihydro-1,4-benzodioxinyl, both being optionally substituted with C₁₋₆alkyl or halo; 2-oxo-2H-1-benzopyranyl and 4-oxo-4H-1-benzo-pyranyl, both being optionally substituted with C₁₋₆alkyl; 3,7-dihydro-1,3-dimethyl-2,6-dioxo-1H-purin-7-yl, optionally substituted with C₁₋₆alkyl; 6-purinyl, and
a bicyclic heterocyclic radical of formula 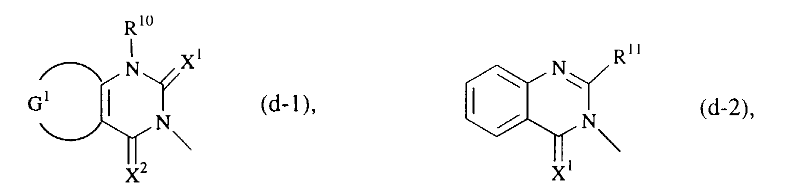 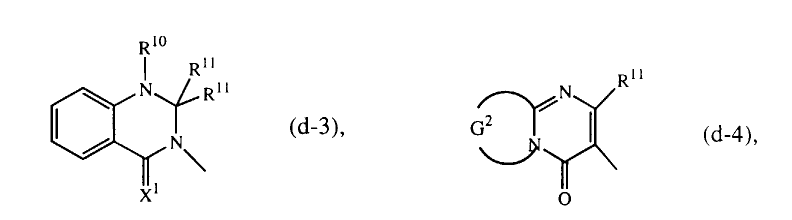 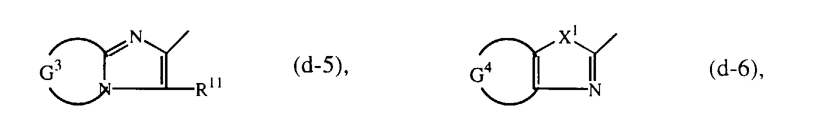 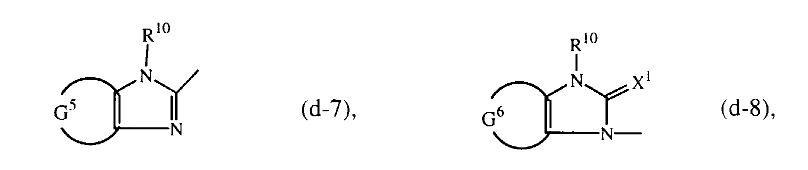 wherein
X¹ and X² each independently are O or S ;
each R¹⁰ and R¹¹ independently is hydrogen or C₁₋₆alkyl;
G¹ is -CH=CH-CH=CH-; -S-CH=CH- or -N=CH-NH- ;
G² is -CH=CH-CH=CH-, -(CH₂)₄-, -S-(CH₂)₂-, -S-(CH₂)₃-, -S-CH=CH-, -CH=CH-O-, -NH-(CH₂)₂-, -NH-(CH₂)₃-, -NH-CH=CH-, -NH-N=CH-CH₂-, -NH-CH=N- or -NH-N=CH- ;
G³ is -CH=CH-CH=CH-, -CH₂-NH-(CH₂)₂-, -S-CH=CH-, -S-(CH₂)₃-, -N=CH-CH=CH-, -CH=N-CH=CH-, -CH=CH-N=CH-, -CH=CH-CH=N-, -N=CH-N=CH- or -CH=N-CH=N- ;
G⁴ is -CH=CH-CH=CH-, -CH₂-NH-(CH₂)₂-, -N=CH-CH=CH-, -CH=N-CH=CH-, -CH=CH-N=CH-, -CH=CH-CH=N-, -N=CH-N=CH- or -CH=N-CH=N-;
G⁵ is -CH=CH-CH=CH-, -N=CH-CH=CH-, -CH=N-CH=CH-, -CH=CH-N=CH-, -CH=CH-CH=N-, -N=CH-N=CH- or -CH=N-CH=N- ; and
G⁶ is -CH=CH-CH=CH-, -N=CH-CH=CH-, -CH=N-CH=CH-, -CH=CH-N=CH-, -CH=CH-CH=N-, -N=CH-N=CH- or -CH=N-CH=N-.

2. A compound according to claim 1 wherein L represents hydrogen, C₁₋₄alkyl, C₁₋₄alkyloxy-carbonyl or a radical of formula -Alk-R³ (c-1), -Alk-Y-R⁴ (c-2) or -Alk-Z¹-C(=O)-Z²-R⁵ (c-3); Alk represents C₁₋₄alkanediyl; R³ represents phenyl, C₁₋₄alkyloxyphenyl or a radical of formula 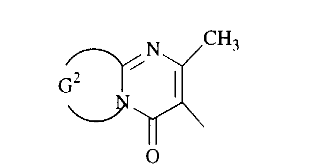 wherein G² represents -CH=CH-CH=CH-, -S-(CH₂)₃-, -S-(CH₂)₂- or -S-CH=CH-; Y represents O or NH; R⁴ represents hydrogen, C₁₋₄alkyl or pyrimidinyl; R⁵ represents C₁₋₄alkyl; Z¹ represents NH; Z² represents O; and X represents O.

3. A compound according to claim 1 wherein R² represents methyl; L represents C₁₋₄alkyl or a radical of formula -Alk-R³ (c-1), -Alk-Y-R⁴ (c-2) or -Alk-Z¹-C(=O)-Z²-R⁵ (c-3); Alk represents C₂₋₄alkanediyl; R³ represents 4-methoxvphenvl or a radical of formula 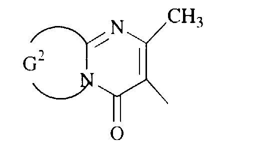 wherein G² represents -CH=CH-CH=CH-, -S-(CH₂)₃-, -S-(CH₂)₂-, -S-CH=CH-; Y represents O or NH; R⁴ represents C₁₋₄alkyl or 2-pyrimidinyl.

4. A compound according to claim 1 wherein L represents methyl or a radical of formula -Alk-R³ (c-1), Alk represents 1,2-ethanediyl and R³ represents 4-methoxyphenyl or a radical of formula 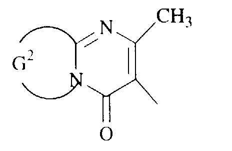 wherein G² represents CH=CH-CH=CH-, -S-(CH₂)₃-, -S-(CH₂)₂- or -S-CH=CH-.

5. An antiallergic composition comprising a pharmaceutically acceptable carrier and as active ingredient an effective antiallergic amount of a compound as claimed in claim 1.

6. A process for preparing a pharmaceutical composition as claimed in claim 5 , characterized in that a therapeutically effective amount of a compound as claimed in any of claims 1 to 4 is intimately mixed with a pharmaceutically acceptable carrier.

7. A compound as claimed in any of claims I to 4 for use as a medicine.

8. A process of preparing a compound as claimed in claim 1, characterized by N-alkylating an intermediate of formula (XV) with an alkylating reagent of formula (XIV) wherein W¹ represents a reactive leaving group, 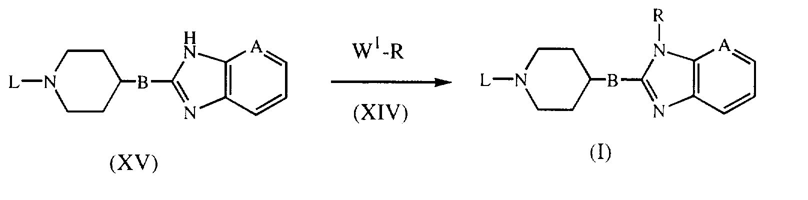 in a reaction-inert solvent in the presence of a base and at an elevated temperature; and optionally,
a) converting a compound of formula (I) wherein L represents benzyl into a compound wherein L represents C₁₋₄alkyloxycarbonyl, 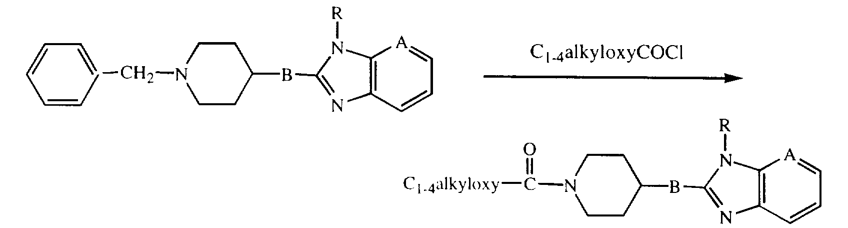 by reaction with a C₁₋₄alkylchloroformate in the presence of a base in a reaction-inert solvent;
b) hydrolyzing a compound of formula (I) wherein L represents C₁₋₄alkyloxycarbonyl in an aqueous acidic or basic medium to a compound of formula (I-e), 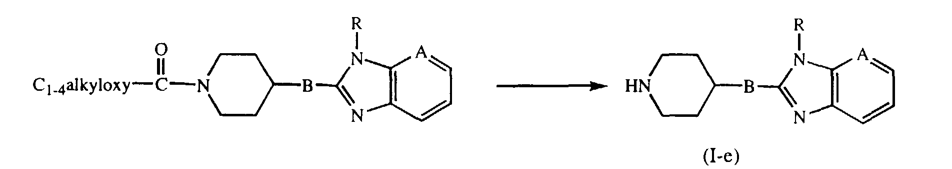
c) N-alkylating a compound of formula (I-e) with an alkylating reagent of formula L¹-W¹ (XVI) in a reaction-inert solvent in the presence of a base, thus preparing a compound of formula 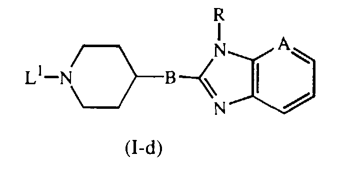
d) reductively N-alkylating a compound of formula (I) with an aldehyde or ketone of formula L²=O (XVII) wherein L² represents a geminal bivalent radical comprising C₃₋₆cycloalkylidene, C₁₋₁₂alkylidene, R³-C₁₋₆alkylidene, R⁴-Y-C₁₋₆alkylidene or R⁵-Z²-C(=O)-Z¹-C₁₋₆alkylidene, 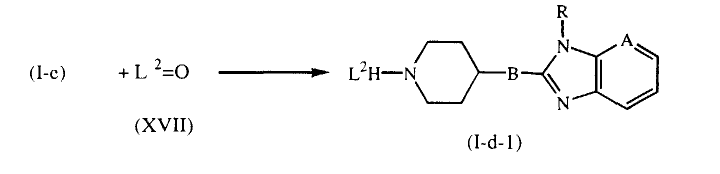 in a reaction-inert sovent in the presence of a reductant;
e) hydrolyzing a compound of formula (I) wherein L represents C₁₋₄alkyloxycarbonylaminoC₂₋₄alkyl in a basic aqueous medium thus yielding a compound wherein L represents an aminoC₂₋₄alkyl radical, 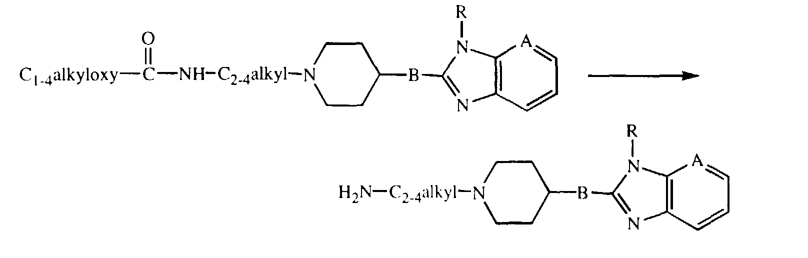
f) alkylating the thus obtained compound wherein L represents aminoC₂₋₄alkyl with a reagent of formula R^{4-a}-W¹ wherein R^{4-a} represents aryl or Het and W¹ represents a reactive leaving group thus yielding a compound of formula 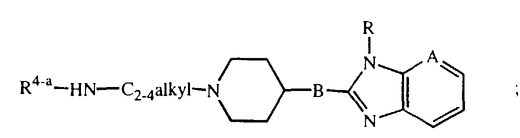 or
g) reacting a compound of formula (I-e) with an epoxide of formula (XXV) wherein R¹² represents hydrogen, C₁₋₄alkyl or R⁶-O-CH₂-, 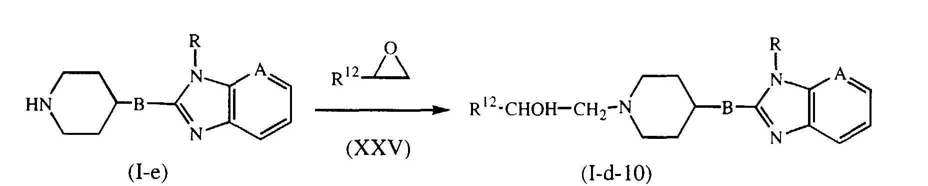 in a reaction-inert solvent; and, if desired, converting the compounds of formula (I) into a salt form by treatment with a pharmaceutically acceptable acid or conversely, converting the salt form into the free base by treatment with alkali; and/or preparing stereochemically isomeric forms thereof.

9. A process of preparing a compound as claimed in claim 1 wherein B is NH,
characterized by
cyclodesulfurizing a thiourea of formula (II-a-1), 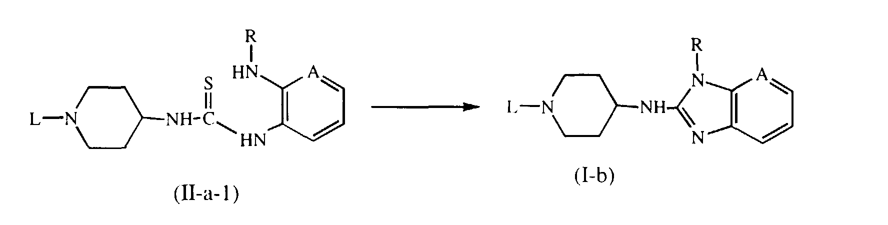 and optionally conducting the group transformations as described in a) - g) of claim 8.

10. Use of a compound as claimed in any one of claims 1 to 4 in the manufacture of a medicament for treating allergic diseases.

## Patentansprüche

1. Verbindung der Formel: 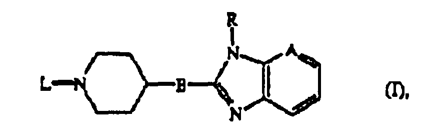 eines ihrer pharmazeutisch unbedenklichen Säureadditionssalze oder eine ihrer stereochemisch isomeren Formen, wobei
-A= für -N= oder -CH= steht;
B für NH oder CH₂ steht;
R für einen Rest der Formel 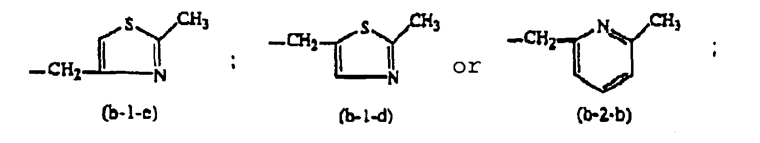 steht;
L für Wasserstoff; C₁₋₁₂Alkyl; C₁₋₆Alkyloxycarbonyl; oder einen Rest der Formel
-Alk-R³ (c-1);
-Alk-Y-R⁴ (c-2);
oder
-Alk-Z¹-C(=O)-Z²-R⁵ (c-3)
steht; wobei
R³ für Het oder gegebenenfalls durch Halogen, c₁₋₆-Alkyl, Hydroxyl oder C₁₋₆Alkyloxy substituiertes Phenyl steht;
R⁴ für Wasserstoff, C₁₋₆Alkyl, Het oder gegebenenfalls durch Halogen, C₁₋₆Alkyl, Hydroxyl oder C₁₋₆Alkyloxy substituiertes Phenyl steht;
R⁵ für Wasserstoff, C₁₋₆Alkyl, Het oder gegebenenfalls durch Halogen, C₁₋₆Alkyl, Hydroxyl oder C₁₋₆Alkyloxy substituiertes Phenyl steht;
Y für O oder NH steht;
Z¹ und Z² jeweils unabhängig voneinander für O oder NH stehen;
jedes Alk unabhängig von den anderen für C₁₋₆Alkandiyl steht;
Het für
Pyridinyl, welches gegebenenfalls durch ein oder zwei Substituenten, die jeweils unabhängig voneinander aus Halogen, Amino, Mono- und Di(C₁₋₆Alkyl)amino, Nitro, Cyano, Aminocarbonyl, C₁₋₆Alkyl, C₁₋₆Alkyloxy, C₁₋₆Alkylthio, C₁₋₆Alkyloxycarbonyl, Hydroxyl, C₁₋₆Alkylcarbonyloxy und Carboxyl ausgewählt sind, substituiert ist; Pyridinyloxid, welches gegebenenfalls durch Nitro substituiert ist; Pyrimidinyl, welches gegebenenfalls durch ein oder zwei Substituenten, die jeweils unabhängig voneinander aus Halogen, Amino, C₁₋₆Alkylamino, Hydroxyl, C₁₋₆Alkyl, C₁₋₆Alkyloxy und C₁₋₆Alkylthio ausgewählt sind, substituiert ist; Pyridazinyl, welches gegebenenfalls durch C₁₋₆Alkyl oder Halogen substituiert ist; Pyrazinyl, welches gegebenenfalls durch Halogen, Amino oder C₁₋₆Alkyl substituiert ist; Thienyl, welches gegebenenfalls durch Halogen oder C₁₋₆Alkyl substituiert ist; Furanyl, welches gegebenenfalls durch Halogen oder C₁₋₆Alkyl substituiert ist; Pyrrolyl, welches gegebenenfalls durch C₁₋₆Alkyl substituiert ist; Thiazolyl, welches gegebenenfalls durch C₁₋₆Alkyl oder C₁₋₆Alkyloxycarbonyl substituiert ist; Imidazolyl, welches gegebenenfalls durch ein oder zwei Substituenten, die jeweils unabhängig voneinander aus C₁₋₆Alkyl und Nitro ausgewählt sind, substituiert ist; Tetrazolyl, welches gegebenenfalls durch C₁₋₆Alkyl substituiert ist; 1,3,4-Thiadiazolyl, welches gegebenenfalls durch C₁₋₆Alkyl oder Amino substituiert ist; 5,6-Dihydro-4H-1,3-thiazin-2-yl, welches gegebenenfalls durch C₁₋₆Alkyl substituiert ist; 4,5-Di-hydrothiazolyl, welches gegebenenfalls durch C₁₋₆Alkyl substituiert ist; Oxazolyl, welches gegebenenfalls durch C₁₋₆Alkyl substituiert ist; 4,5-Dihydro-5-oxo-1H-tetrazolyl, welches gegebenenfalls durch C₁₋₆Alkyl substituiert ist; 1,4-Dihydro-2,4-dioxo-3(2H)-pyrimidinyl, welches gegebenenfalls durch C₁₋₆Alkyl substituiert ist; 3,4-Dihydro-4-oxopyrimidinyl oder 4,5-Dihydro-4-oxopyrimidinyl, wobei beide Reste gegebenenfalls durch bis zu 3 Substituenten, ausgewählt aus C₁₋₆Alkyl, Amino, C₁₋₆Alkylaminocarbonylamino, Arylaminocarbonylamino, ArylC₁₋₆alkylamino und C₁₋₆Alkylamino, substituiert sind; 2,3-Dihydro-3-oxopyridazinyl; 2-oxo-3-oxazolidinyl; Pyrrolidinyl; Piperidinyl; Morpholinyl; Thiomorpholinyl; Dioxanyl, welches gegebenenfalls durch C₁₋₆Alkyl substituiert ist; Indolyl, welches gegebenenfalls durch Hydroxyl oder C₁₋₆Alkyl substituiert ist; Chinolyl, welches gegebenenfalls durch Hydroxyl oder C₁₋₆Alkyl substituiert ist; Chinazolinyl, welches gegebenenfalls durch Hydroxyl oder C₁₋₆Alkyl substituiert ist; Chinoxalinyl, welches gegebenenfalls durch C₁₋₆Alkyl substituiert ist; Phthalazinyl, welches gegebenenfalls durch Halogen substituiert ist; 1,3-Dioxo-1H-isoindol-2(3H)-yl; 2,3-Dihydro-3-oxo-4H-benzoxazinyl und 2,3-Dihydro-1,4-benzodioxinyl, wobei beide Reste gegebenenfalls durch C₁₋₆Alkyl oder Halogen substituiert sind; 2-Oxo-2H-1-benzopyranyl und 4-Oxo-4H-1-benzopyranyl, wobei beide Reste gegebenenfalls durch C₁₋₆Alkyl substituiert sind; 3,7-Dihydro-1,3-dimethyl-2,6-dioxo-1H-purin-7-yl, welches gegebenenfalls durch C₁₋₆Alkyl substituiert ist; 6-Purinyl, und
einen bizyklischen heterozyklischen Rest der Formel 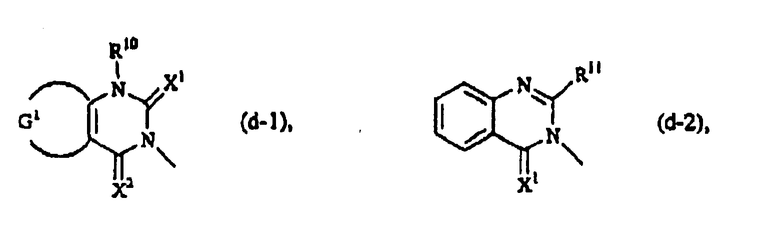 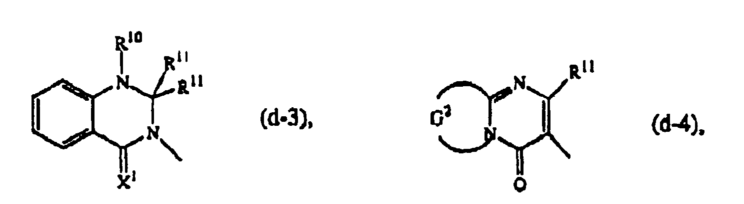 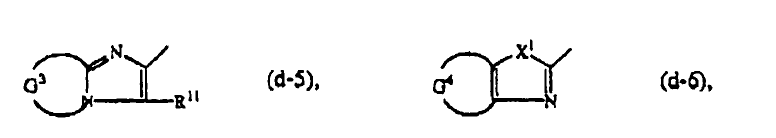 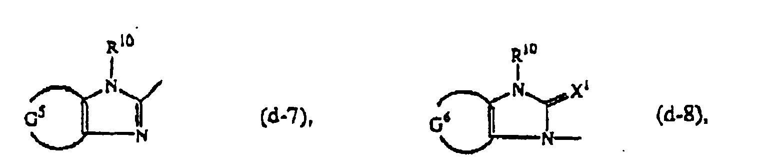 steht,
wobei
X¹ und X² jeweils unabhängig voneinander für O oder S stehen;
jedes R¹⁰ und R¹¹ unabhängig voneinander für Wasserstoff oder C₁₋₆Alkyl steht;
G¹ für -CH=CH-CH=CH; -S-CH=CH- oder -N=CH-NH- steht;
G² für -CH=CH-CH=CH, -(CH₂)₄-, -S-(CH₂)₂-,-S-(CH₂)₃-, -S-CH=CH-, -CH=CH-O-, -NH- (CH₂)₂-, -NH- (CH₂)₃-, -NH-CH=CH-, -NH-N=CH-CH₂-, -NH-CH=N- oder -NH-N=CH- steht;
G³ für -CH=CH-CH=CH-, -CH2-NH-(CH2)2-, -S-CH=CH-, -S-(CH₂)₃-, -N=CH-CH=CH-, -CH=N-CH=CH-, -CH=CH-N=CH-, -CH=CH-CH=N-, -N=CH-N=CH- oder -CH=N-CH=N- steht;
G⁴ für -CH=CH-CH=CH-, -CH₂-NH-(CH₂)₂-, -N=CH-CH=CH-, -CH=N-CH=CH-, -CH=CH-N=CH-, -CH=CH-CH=N-, -N=CH-N=CH- oder -CH=N-CH=N- steht;
G⁵ für -CH=CH-CH=CH-, -N=CH-CH=CH-, -CH=N-CH=CH-, -CH=CH-N=CH-, -CH=CH-CH=N-, -N=CH-N=CH- oder -CH=N-CH=N- steht und
G⁶ für -CH=CH-CH=CH-, -N=CH-CH=CH-, -CH=N-CH=CH-, -CH=CH-N=CH-, -CH=CH-CH=N-, -N=CH-N=CH- oder -CH=N-CH=N- steht.

2. Verbindung gemäß Anspruch 1, wobei L für Wasserstoff, C₁₋₄Alkyl, C₁₋₄Alkyloxy-Carbonyl oder einen Rest der Formel -Alk-R³ (c-1), -Alk-Y-R⁴ (c-2) oder -Alk-Z¹-C(=O)-Z²-R⁵ (c-3) steht; Alk für C₁₋₄Alkandiyl steht; R³ für Phenyl, C₁₋₄Alkyloxyphenyl oder einen Rest der Formel 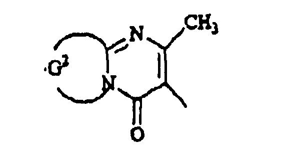 steht, wobei G² für -CH=CH-CH=CH-, -S-(CH₂)₃-, -S-(CH₂)₂-, oder -S-CH=CH- steht; Y für O oder NH steht; R⁴ für Wasserstoff, C₁₋₄Alkyl oder Pyrimidinyl steht; R⁵ für C₁₋₄Alkyl steht; Z¹ für NH steht; Z² für O steht; und X für O steht.

3. Verbindung gemäß Anspruch 1, wobei R² für Methyl steht; L für C₁₋₄Alkyl oder einen Rest der Formel -Alk-R³ (c-1), -Alk-Y-R⁴ (c-2) oder -Alk-Z¹-C(=O)-Z²-R⁵ (c-3) steht; Alk für C₂₋₄Alkandiyl steht; R³ für 4-Methoxyphenyl oder einen Rest der Formel 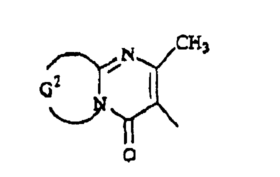 steht,
wobei G² für -CH=CH-CH=CH-, -S-(CH₂)₃-, -S- (CH₂)₂-, -S-CH=CH- steht; Y für O oder NH steht; R⁴ für C₁₋₄Alkyl oder 2-Pyrimidinyl steht.

4. Verbindung gemäß Anspruch 1, wobei L für Methyl oder einen Rest der Formel -Alk-R³ (c-1) steht, Alk für 1,2-Ethandiyl steht und R³ für 4-Methoxyphenyl oder einen Rest der Formel 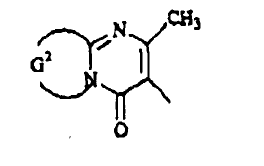 steht,
wobei G² für -CH=CH-CH=CH-, -S-(CH₂)₃-, -S-(CH₂)₂- oder -S-CH=CH- steht.

5. Antiallergische Zusammensetzung, welche einen pharmazeutisch unbedenklichen Trägerstoff und als Wirkstoff eine antiallergisch wirksame Menge einer Verbindung gemäß Anspruch 1 enthält.

6. Verfahren zur Herstellung einer pharmazeutischen Zusammensetzung gemäß Anspruch 5, dadurch gekennzeichnet, daß man eine therapeutisch wirksame Menge einer Verbindung gemäß einem der Ansprüche 1 bis 4 innig mit einem pharmazeutisch unbedenklichen Trägerstoff mischt.

7. Verbindung gemäß einem der Ansprüche 1 bis 4 zur Verwendung als Medikament.

8. Verfahren zur Herstellung einer Verbindung gemäß Anspruch 1, dadurch gekennzeichnet, daß man ein Zwischenprodukt der Formel (XV) mit einem Alkylierungsmittel der Formel (XIV), wobei W¹ für eine reaktive Abgangsgruppe steht, 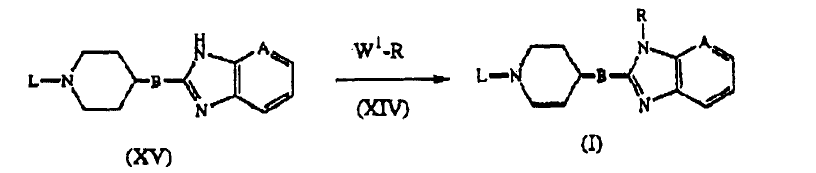 in einem reaktionsinerten Lösungsmittel in Gegenwart einer Base und bei erhöhter Temperatur N-alkyliert; und gegebenenfalls
a) eine Verbindung der Formel (I), wobei L für Benzyl steht, in eine Verbindung, in welcher L für C₁₋₄Alkyloxycarbonyl steht, umwandelt 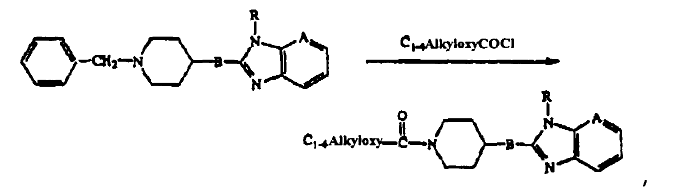 indem man sie mit einem Chlorameisensäure-C₁₋₄Alkylester in Gegenwart einer Base in einem reaktionsinerten Lösungsmittel umsetzt;
b) eine Verbindung der Formel (I), wobei L für C₁₋₄Alkyloxycarbonyl steht, in saurem oder basischem wäßrigem Medium zu einer Verbindung der Formel (I-e) hydrolysiert, 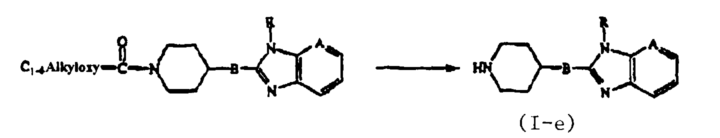
c) eine Verbindung der Formel (I-e) mit einem Alkylierungsmittel der Formel L¹-W¹ (XVI) in einem reaktionsinerten Lösungsmittel in Gegenwart einer Base N-alkyliert und so eine Verbindung der Formel 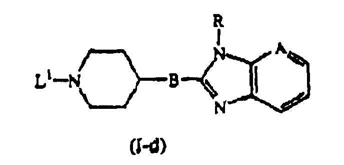 herstellt;
d) eine Verbindung der Formel (I) mit einem Aldehyd oder Keton der Formel L²=O (XVII), wobei L² für einen geminalen bivalenten Rest aus der Gruppe C₃₋₆Cycloalkyliden, C₁₋₁₂Alkyliden, R³-C₁₋₆Alkyliden, R⁴-Y-C₁₋₆Alkyliden oder R⁵-Z²-C(=O)-Z¹-C₁₋₆Alkyliden steht, in einem reaktionsinerten Lösungsmittel in Gegenwart eines Reduktionsmittels reduktiv N-alkyliert 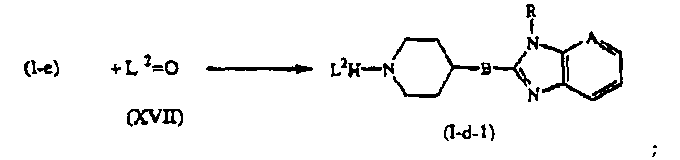
e) eine Verbindung der Formel (I), wobei L für C₁₋₄AlkyloxycarbonylaminoC₂₋₄Alkyl steht, in basischem wäßrigen Medium hydrolisiert und so eine Verbindung erhält, in der L für einen AminoC₂₋₄Alkyl-Rest steht, 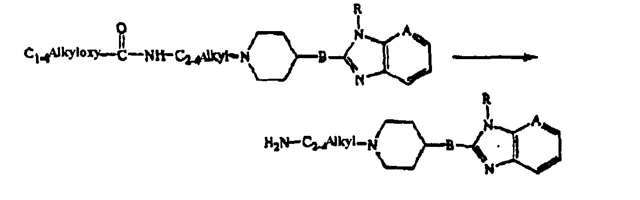
f) die so erhaltene Verbindung, in der L für AminoC₂₋₄Alkyl steht, mit einem Reagens der Formel R^{4-a}-W¹, wobei R^{4-a} für Aryl oder Het und W¹ für eine reaktive Abgangsgruppe steht, alkyliert und so eine Verbindung der Formel 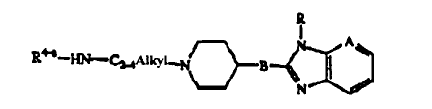 erhält; oder
g) eine Verbindung der Formel (I-e) mit einem Epoxid der Formel (XXV), wobei R¹² für Wasserstoff, C₁₋₄Alkyl oder R⁶-O-CH₂- steht, in einem reaktionsinerten Lösungsmittel reagieren läßt, 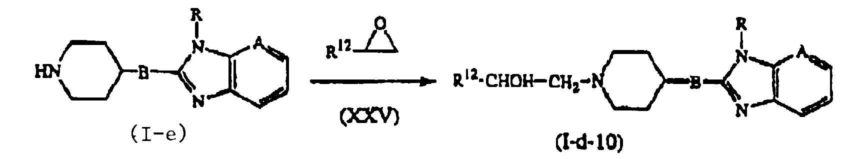 und, falls gewünscht, die Verbindungen der Formel (I) durch Behandlung mit einer pharmazeutisch unbedenklichen Säure in ein Salz oder umgekehrt die Salzform durch Behandlung mit Alkali in die freie Base umwandelt; und/oder stereochemisch isomere Formen davon herstellt.

9. Verfahren zur Herstellung einer Verbindung gemäß Anspruch 1, in der B für NH steht, dadurch charakterisiert, daß
man einen Thioharnstoff der Formel (II-a-1) 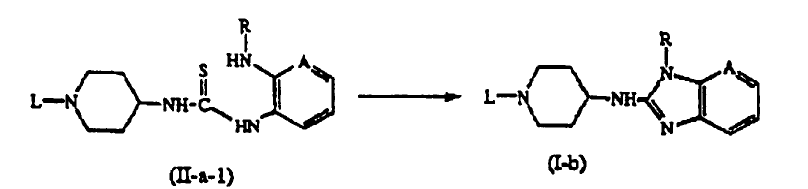 cyclodesulphurisiert und gegebenenfalls die in Anspruch 8 unter a)-g) beschriebenen Gruppentransformationen durchführt.

10. Verwendung einer Verbindung gemäß einem der Ansprüche 1 bis 4 zur Herstellung eines Medikaments zur Behandlung von allergischen Erkrankungen.

## Revendications

1. Composé de formule: 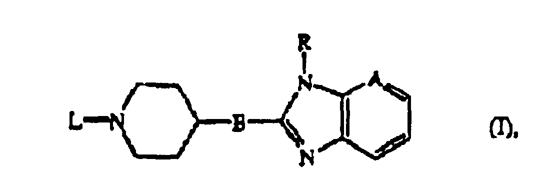 sel d'addition pharmaceutiquement acceptable à un acide ou forme stéréochimiquement isomère de celui-ci, où
-A= est -N= ou -CH=;
B représente NH ou CH₂;
R est un radical de formule; 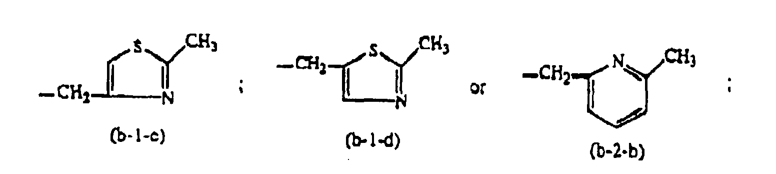
L est un hydrogène; un alkyle en C₁₋₁₂; un C₁₋₆-alkyloxycarbonyle; ou un radical de formules
-Alk-R³ (c-1);
-Alk-Y-R⁴ (c-2);
ou
-Alk-Z¹-C(=O)-Z²-R⁵ (c-3);
dans lesquelles
R³ est Hét ou un phényle éventuellement substitué par un halogéno, un alkyle en C₁₋₆, un hydroxy ou alkyloxy en C₁₋₆;
R⁴ est un hydrogène, un alkyle en C₁₋₆, Hét ou un phényle éventuellement substitué par un halogéno, un alkyle en C₁₋₆, un hydroxy ou un alkyloxy en C₁₋₆;
R⁵ est un hydrogène, un alkyle en C₁₋₆, Hét ou un phényle éventuellement substitué par un halogéno, un alkyle en C₁₋₆, un hydroxy ou un alkyloxy en C₁₋₆;
Y est O ou NH;
Z¹ et Z² sont chacun indépendamment O ou NH;
chaque Alk est indépendamment un C₁₋₆-alcanediyle;
Hét est un pyridinyle, éventuellement substitué à un ou deux substituants chacun choisi indépendamment parmi un halogéno, un amino, un mono- et un di(C₁₋₆-alkyl)amino, un nitro, un cyano, un aminocarbonyle, un alkyle en C₁₋₆, un alkyloxy en C₁₋₆, un alkylthio en C₁₋₆, un C₁₋₆-alkyloxycarbonyle, un hydroxy, un C₁₋₆-alkylcarbonyloxy et un carboxyle; un pyridinyloxyde, éventuellement substitué par un nitro; un pyrimidinyle, éventuellement substitué par un ou deux substituants chacun choisi indépendamment parmi un halogéno, un amino, un alkylamino en C₁₋₆, un hydroxy, un alkyle en C₁₋₆, un alkyloxy en C₁₋₆ et alkylthio en C₁₋₆; un pyridazinyle, éventuellement substitué par un alkyle en C₁₋₆ ou un halogéno; un pyrazinyle, éventuellement substitué par un halogéno, un amino ou un alkyle en C₁₋₆; un thiényle, éventuellement substitué par un halogéno ou un alkyle en C₁₋₆; un furanyle, éventuellement substitué par un halogéno ou un alkyle en C₁₋₆; un pyrrolyle, éventuellement substitué par un alkyle en C₁₋₆; un thiazolyle, éventuellement substitué par un alkyle en C₁₋₆ ou un C₁₋₆-alkyloxycarbonyle; un imidazolyle, éventuellement substitué par un ou deux substituants chacun choisi indépendamment parmi un alkyle en C₁₋₆ et un nitro; un tétrazolyle, éventuellement substitué par un alkyle en C₁₋₆; un 1,3,4-thiadiazolyle, éventuellement substitué par un alkyle en C₁₋₆ ou un amino; un 5,6-dihydro-4H-1,3-thiazin-2-yle, éventuellement substitué par un alkyle en C₁₋₆; un 4,5-dihydrothiazolyle, éventuellement substitué par un alkyle en C₁₋₆; un oxazolyle, éventuellement substitué par un alkyle en C₁₋₆; un 4,5-dihydro-5-oxo-1H-tétrazolyle, éventuellement substitué par un alkyle en C₁₋₆; un 1,4-dihydro-2,4-dioxo-3(2H)-pyrimidinyle, éventuellement substitué par un alkyle en C₁₋₆; un 3,4-dihydro-4-oxopyrimidinyle ou un 4,5-dihydro-4-oxopyrimidinyle, les deux radicaux étant éventuellement substitués par jusqu'à 3 substituants choisis par un alkyle en C₁₋₆, un amino, un C₁₋₆-alkylaminocarbonylamino, un arylaminocarbonylamino, un aryl- C₁₋₆-alkylamino et un alkylamino en C₁₋₆; un 2,3-dihydro-3-oxopyridazinyle; un 2-oxo-3-oxazolidinyle; un pyrrolidinyle; un pipéridinyle; un morpholinyle; un thiomorpholinyle; un dioxanyle, éventuellement substitué par un alkyle en C₁₋₆; un indolyle, éventuellement substitué par un hydroxy ou un alkyle en C₁₋₆; un quinolinyle, éventuellement substitué par un hydroxy ou un alkyle en C₁₋₆; un quinazolinyle, éventuellement substitué par un hydroxy ou alkyle en C₁₋₆; un quinoxalinyle, éventuellement substitué par un alkyle en C₁₋₆; un phthalazinyle, éventuellement substitué par un halogéno; un 1,3-dioxo-1H-iso-indol-2(3H)-yle; un 2,3-dihydro-3-oxo-4H-benzoxazinyle et un 2,3-dihydro-1,4-benzodioxinyle, les deux étant éventuellement substitués par un alkyle en C₁₋₆ ou un halogéno; un 2-oxo-2H-1-benzopyranyle et un 4-oxo-4H-1-benzopyranyle, les deux étant éventuellement substitués par un alkyle en C₁₋₆; un 3,7-dihydro-1,3-diméthyle-2,6-dioxo-1H-purin-7-yle, éventuellement substitué par un alkyle en C₁₋₆; un 6-purinyle, et un radical hétérocyclique bicyclique de formules 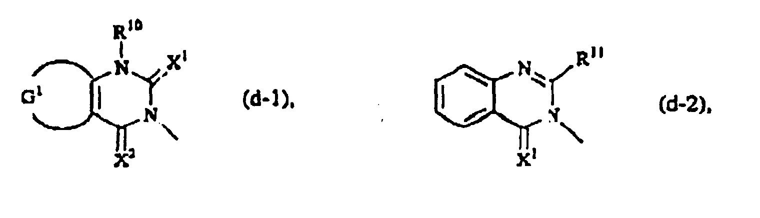 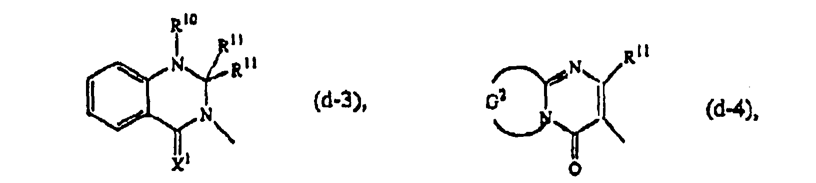 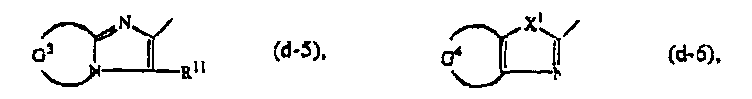 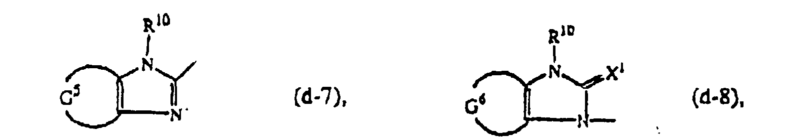 dans lesquelles
X¹ et X² sont chacun indépendamment O ou S;
chaque R¹⁰ et R¹¹ est indépendamment un hydrogène ou en alkyle en C₁₋₆;
G¹ est -CH=CH-CH=CH-; -S-CH=CH- ou -N=CH-NH-;
G² est -CH=CH-CH=CH-, -(CH₂)₄-, -S-(CH₂)₂-, -S-(CH₂)₃-, -S-CH=CH-, -CH=CH-O-, -NH-(CH₂)₂-, NH-(CH₂)₃-, -NH-CH=CH-, -NH-N=CH-CH2 -, -NH-CH=N- ou -NH-N=CH-;
G³ est -CH=CH-CH=CH-, -CH₂-NH-(CH₂)₂-, -S-CH=CH-, -S-(CH₂)₃-, -N=CH-CH=CH-, -CH=N-CH=CH-, -CH=CH-N=CH-, -CH=CH-CH=N-, -N=CH-N=CH- ou -CH=N-CH=N-;
G⁴ est -CH=CH-CH=CH-, -CH₂-NH-(CH₂)₂-, -N=CH-CH=CH-, -CH=N-CH=CH-, -CH=CH-N=CH-, -CH=CH-CH=N-, -N=CH-N=CH- ou -CH=N-CH=N-;
G⁵ est -CH=CH-CH=CH-, -N=CH-CH=CH-, -CH=N-CH=CH-, -CH=CH-N=CH-, -CH=CH-CH=N-, -N=CH-N=CH- ou -CH=N-CH=N-; et
G⁶ est -CH=CH-CH=CH-, -N=CH-CH=CH-, -CH=N-CH=CH-, -CH=CH-N=CH-, -CH=CH-CH=N-, -N=CH-N=CH- ou -CH=N-CH=N-.

2. Composé selon la revendication 1, dans lequel L représente un hydrogène, un alkyle en C₁₋₄, un C₁₋₄- alkyloxy-carbonyle ou un radical de formule Alk- R³ (c-1), -Alk-Y-R⁴ (c-2) ou -Alk-Z¹-C(=O)-Z²-R⁵ (c-3); Alk représente un C₁₋₄-alcanediyle; R³ représente un phényle, un C₁₋₄-alkyloxyphényle ou un radical de formule 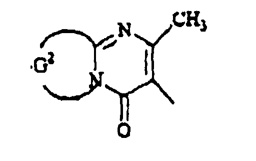 dans laquelle G² représente -CH=CH-CH=CH-, -S-(CH₂)₃-, -S-(CH₂)₂ ou -S-CH=CH-;
Y représente O ou NH; R⁴ représente un hydrogène, un alkyle en C₁₋₄ ou un pyrimidinyle; R⁵ représente un alkyle en C₁₋₄; Z¹ représente NH; Z² représente O; et X représente O.

3. Composé selon la revendication 1, dans lequel R2 représente un méthyle; L représente un alkyle en C₁₋₄ ou un radical de formule -Alk-R³ (c-1), Alk-Y-R⁴ (c-2) ou -Alk-Z¹-C(=O)-Z²-R⁵ (c-3); Alk représente un C₂₋₄alcanediyle; R³ représente un 4-méthoxyphényle ou un radical de formule 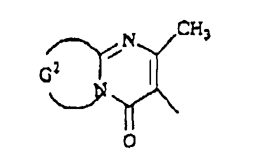 dans laquelle G² représente -CH=CH-CH=CH-, -S-(CH₂)₃-, -S-(CH₂)₂-, -S-CH=CH-;
Y représente O ou NH; R⁴ représente un alkyle en C₁₋₄ ou un 2-pyrimidinyle.

4. Composé selon la revendication 1, dans lequel L représente un méthyle ou un radical de formule -Alk-R³ (c-1), Alk représente un 1,2-éthanediyle et R³ représente un 4-méthoxyphényle ou un radical de formule 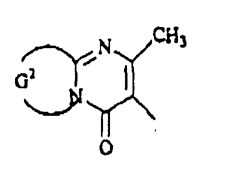 dans laquelle G² représente -CH=CH-CH=CH-, -S-(CH₂)₃-, -S-(CH₂)₂- ou -S-CH=CH-

5. Composition antiallergique comprenant un support pharmaceutiquement acceptable et, en tant qu'ingrédient actif, une quantité antiallergique efficace d'un composé selon la revendication 1.

6. Procédé de préparation d'une composition pharmaceutique selon la revendication 5, caractérisé en ce qu'une quantité thérapeutiquement efficace d'un composé selon l'une quelconque des revendications 1 à 4 est mélangée intimement avec un support pharmaceutiquement acceptable.

7. Composé selon l'une quelconque des revendications 1 à 4, destiné à être utilisé en tant que médicament.

8. Procédé de préparation d'un composé selon la revendication 1, caractérisé par la N-alkylation d'un intermédiaire de formule (XV) avec un réactif d'alkylation de formule (XIV) dans laquelle W¹ représente un groupe partant réactif, 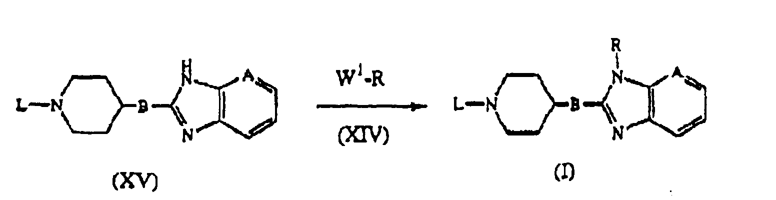 dans un solvant inerte vis-à-vis de la réaction en présence d'une base et à une température élevée; et éventuellement,
a) la transformation d'un composé de formule (I) dans laquelle L représente un benzyle en un composé dans lequel L représente un C₁₋₄-alkyloxycarbonyle, 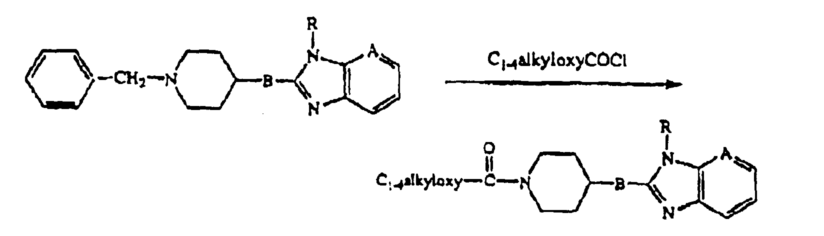 par réaction avec un C₁₋₄-alkylchloroformate en présence d'une base dans un solvant inerte vis-à-vis de la réaction;
b) l'hydrolyse d'un composé de formule (I) dans laquelle L représente un C₁₋₄-alkyloxycarbonyle dans un milieu aqueux, acide ou basique pour donner un composé de formule (I-e), 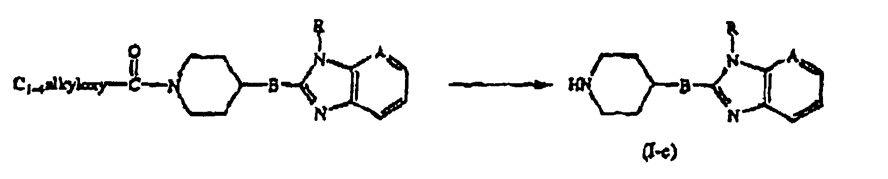
c) la N-alkylation d'un composé de formule (I-e) avec un réactif d'alkylation de formule L¹-W¹ (XVI) dans un solvant inerte vis-à-vis de la réaction en présence d'une base, ce qui permet ainsi de préparer un composé de formule 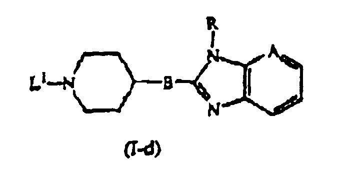
d) la N-alkylation réductive d'un composé de formule (I) avec un aldéhyde ou une cétone de formule L²=O (XVII) dans laquelle L² représente un radical bivalent géminal comprenant un C₃₋₆-cycloalkylidène, un C₁₋₁₂-alkylidène, un R³-C₁₋₆-alkylidène, un R⁴-Y-C₁₋₆-alkylidène ou un R⁵-Z²-C(=O)-Z¹-C₁₋₆-alkylidène, 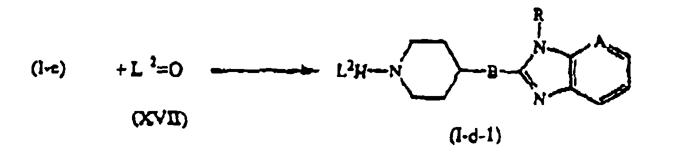 dans un solvant inerte vis-à-vis de la réaction en présence d'un réducteur;
e) l'hydrolyse d'un composé de formule (I) dans laquelle L représente un C₁₋₄-alkyloxycarbonylamino-C₂₋₄-alkyle dans un milieu aqueux basique, en donnant ainsi lieu à un composé dans lequel L représente un radical amino-C₂₋₄-alkyle, 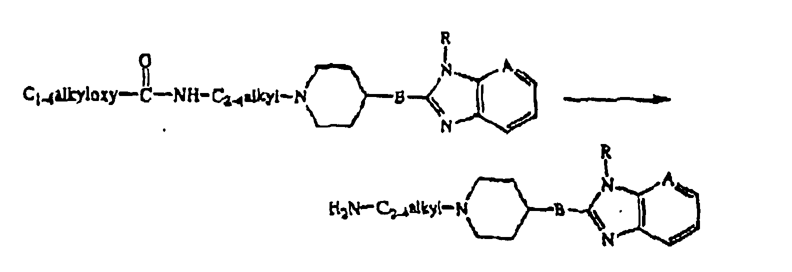
f) l'alkylation du composé ainsi obtenu dans lequel L représente un amino-C₂₋₄-alkyle avec un réactif de formule R^{4-a}-W¹ dans laquelle R^{4-a} représente un aryle ou Hét et W¹ représente un groupe partant réactif, en donnant ainsi lieu à un composé de formule 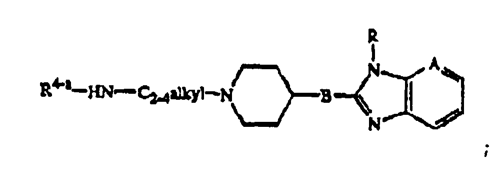 ou
g) la réaction d'un composé de formule (I-e) avec un époxyde de formule (XXV) dans laquelle R¹² représente un hydrogène, un alkyle en C₁₋₄ ou R⁶-O-CH₂-, 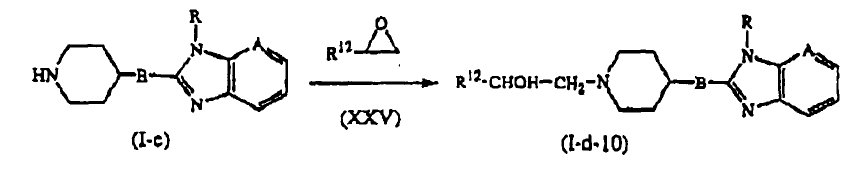 dans un solvant inerte vis-à-vis de la réaction; et, si on le souhaite, la transformation des composés de formule (I) en une forme de sel par traitement avec un acide pharmaceutiquement acceptable ou inversement, la transformation de la forme de sel en base libre par traitement avec un alcali; et/ou la préparation de formes stéréochimiquement isomères de ceux-ci.

9. Procédé de préparation d'un composé selon la revendication 1, dans lequel B est NH, caractérisé par la cyclodésulfuration d'une thio-urée de formule (II-a-1), 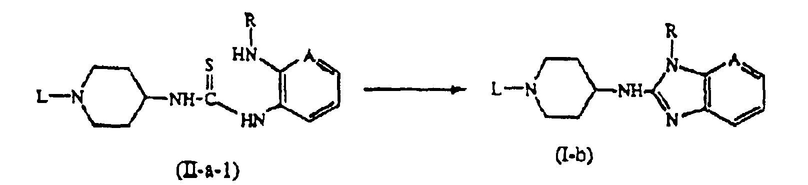 et éventuellement la réalisation des transformations de groupes telles que décrites dans a) - g) de la revendication 8.

10. Utilisation d'un composé selon l'une quelconque des revendications 1 à 4 pour l'élaboration d'un médicament destiné à traiter des maladies allergiques.
